# EUROPEAN PATENT SPECIFICATION

(11) **EP 3 080 302 B1**
(45) Date of publication and mention of the grant of the patent: **16.09.2020**
(21) Application number: 14869706.3
(22) Date of filing: 10.12.2014
(51) Int. Cl.: C12Q 1/6874, C12Q 1/6881

(54) **METHODS AND PROBES FOR IDENTIFYING GENE ALLELES**
VERFAHREN UND SONDEN ZUR IDENTIFIKATION VON GEN-ALLELEN
PROCÉDÉS ET SONDES POUR IDENTIFIER DES ALLÈLES GÉNIQUES

(30) Priority: 10.12.2013 AU 2013904801
(43) Date of publication of application: 19.10.2016
(60) Divisional application: 20178680.3
(73) Proprietor: Conexio Genomics Pty Ltd, East Fremantle, Western Australia 6158 (AU)
(72) Inventor: SAYER, David, Charles, East Fremantle, Western Australia 6158 (AU); DESANTIS, Dianne, Mt Hawthorn, Western Australia 6016 (AU)
(74) Representative: Boult Wade Tennant LLP
(86) International application number: PCT/AU2014/001114
(87) International publication number: WO 2015/085350

(56) References cited:
- EP-A2- 0 892 069
- WO-A1-97/23645
- WO-A1-99/07883
- WO-A1-2009/061734
- WO-A1-2010/151416
- WO-A1-2012/171990
- WO-A2-97/20197
- WO-A2-2005/108624
- WO-A2-2014/116729
- US-A1- 2004 197 775
- DATABASE Geneseq [Online] 11 June 2007 (2007-06-11), "Human HLA-A intron 1 genomic DNA from allele A*3002.", XP002770312, retrieved from EBI accession no. GSN:AAV46064 Database accession no. AAV46064
- DATABASE EMBL [Online] 16 November 2000 (2000-11-16), "Sequence 332 from Patent WO0061795.", XP002770313, retrieved from EBI accession no. EM_PAT:AX038575 Database accession no. AX038575
- DATABASE Geneseq [Online] 11 June 2007 (2007-06-11), "Histocompatibility locus antigen A intron 3 SEQ ID NO:138.", XP002770314, retrieved from EBI accession no. GSN:AAX37982 Database accession no. AAX37982
- DATABASE EMBL [Online] 16 January 2001 (2001-01-16), "RC3-CI0162-201100-022-f06 CI0162 Homo sapiens cDNA, mRNA sequence.", XP002770315, retrieved from EBI accession no. EM_EST:BF812144 Database accession no. BF812144
- DATABASE EMBL [Online] 8 July 1993 (1993-07-08), "Human MHC class I HLA-A gene, partial intron 3 (HLA-A3).", XP002770316, retrieved from EBI accession no. EM_STD:L15370 Database accession no. L15370
- DATABASE EMBL [Online] 8 July 1993 (1993-07-08), "Human MHC class I HLA-A gene, partial intron 3 (HLA-A1).", XP002770317, retrieved from EBI accession no. EM_STD:L15368 Database accession no. L15368
- LIND, C. ET AL.: 'Complete genomic sequencing of HLA-C*04:09n using next- generation sequencing technology' HUMAN IMMUNOLOGY vol. 71, no. SUPPL., September 2010, page S129, XP027281341
- DE SANTIS, D. ET AL.: '16 (th) IHIW : review ofCHLA typing by NGS' INTERNATIONAL JOURNAL OF IMMUNOGENETICS vol. 40, no. 1, February 2013, pages 72 - 76, XP055349624
- GABRIEL C ET AL: "Rapid high-throughput human leukocyte antigen typing by massively parallel pyrosequencing for high-resolution allele identification", HUMAN IMMUNOLOGY, NEW YORK, NY, US, vol. 70, no. 11, 1 November 2009 (2009-11-01), pages 960-964, XP026698160, ISSN: 0198-8859, DOI: 10.1016/J.HUMIMM.2009.08.009 [retrieved on 2009-08-23]

### SEQUENCE LISTING

<110> Conexio Genomics Pty Ltd
<120> Methods and Probes for Identifying Gene Alleles
<130> P95258.PCT
<150> AU 2013904801
   <151> 2013-12-10
<160> 71
<170> PatentIn version 3.5
<210> 1
   <211> 74
   <212> DNA
   <213> Artificial Sequence
<220>
   <223> Oligonucleotide Capture Probe 01-I1.1 (01 = allele group; I1 = intron number 1; .1 = probe one for intron)
<400> 1
   cgcctctgcg gggagaagca aggggccctc ctggcggggg cgcaggaccg ggggagccgc 60
   gccgggagga gggt 74
<210> 2
   <211> 74
   <212> DNA
   <213> Artificial Sequence
<220>
   <223> Oligonucleotide capture probe 01-I2.1 (01 = allele group; I2 = intron number 2; .1 = probe one for intron)
<400> 2
   cacagtctcc gggtccgaga tccaccccga agccgcggga ctccgagacc cttgtcccgg 60
   gagaggccca ggcg 74
<210> 3
   <211> 87
   <212> DNA
   <213> Artificial Sequence
<220>
   <223> Oligonucleotide capture probe 01-I3.1 (01 = allele group; I3 = intron number 3; .1 = probe one for intron)
<400> 3
   gtaccagggg ccacggggcg cctccctgat cgcctataga tctcccgggc tggcctccca 60
   caaggagggg agacaattgg gaccaac 87
<210> 4
   <211> 82
   <212> DNA
   <213> Artificial Sequence
<220>
   <223> Oligonucleotide capture probe 01-I3.2 (01 = allele group; I3 = intron number 3; .2 = probe two for intron)
<400> 4
   tatcaccctc cctctggtcc tgagggagag gaatcctcct gggtttccag atcctgtacc 60
   agagagtgac tctgaggttc cg 82
<210> 5
   <211> 80
   <212> DNA
   <213> Artificial Sequence
<220>
   <223> Oligonucleotide capture probe 01-I3.3 (01 = allele group; I3 = intron number 3; .3 = probe three for intron)
<400> 5
   acacaattaa gggataaaat ctctgaagga gtgacgggaa gacgatccct cgaatactga 60
   tgagtggttc cctttgacac 80
<210> 6
   <211> 86
   <212> DNA
   <213> Artificial Sequence
<220>
   <223> Oligonucleotide capture probe 01-I3.4 (01 = allele group; I3 = intron number 3; .4 = probe four for intron)
<400> 6
   ttgggcccgt gacttttcct ctcaggcctt gttctctgct tcacactcaa tgtgtgtggg 60
   ggtctgagtc cagcacttct gagtct 86
<210> 7
   <211> 90
   <212> DNA
   <213> Artificial Sequence
<220>
   <223> Oligonculeotide capture probe 01-I4.1 (01 = allele group; I4 = intron number 4; .1 = probe one for intron)
<400> 7
   atgggggtgt catgtctctt agggaaagca ggagcctctc tggagacctt tagcagggtc 60
   agggcccctc accttcccct cttttcccag 90
<210> 8
   <211> 79
   <212> DNA
   <213> Artificial Sequence
<220>
   <223> Oligonucleotide capture probe 01-I5.1 (01 = allele group; I5 = intron number 5; .1 = probe one for intron)
<400> 8
   cttccacaat catgggccga cccagcctgg gccctgtgtg ccagcactta ctcttttgta 60
   aagcacctgt taaaatgaa 79
<210> 9
   <211> 130
   <212> DNA
   <213> Homo sapiens
<400> 9
   gtgagtgcgg ggtcgggagg gaaaccgcct ctgcggggag aagcaagggg ccctcctggc        60
   gggggcgcag gaccggggga gccgcgccgg gaggagggtc gggcaggtct cagccactgc       120
   tcgcccccag 130
<210> 10
   <211> 127
   <212> DNA
   <213> Artificial
<220>
   <223> A-3-1.1 HLA-A 3' untranslated region capture probe sequence
<400> 10
   gacagctgcc ttgtgtggga ctgagaggca agagttgttc ctgcccttcc ctttgtgact 60
   tgaagaaccc tgactttgtt tctgcaaagg cacctgcatg tgtctgtgtt cgtgtaggca 120
   taatgtg 127
<210> 11
   <211> 120
   <212> DNA
   <213> Artificial Sequence
<220>
   <223> A-5-1.1 HLA-A 5' untranslated region capture probe sequence
<400> 11
   cagggcgaag tcccagggcc ccaggcgtgg ctctcagggt ctcaggcccc gaaggcggtg 60
   tatggattgg ggagtcccag ccttggggat tccccaactc cgcagtttct tttctccctc 120
<210> 12
   <211> 120
   <212> DNA
   <213> Artificial Sequence
<220>
   <223> A-5-2.1 HLA-A 5' untranslated region capture probe sequence
<400> 12
   cctggatact cacgacgcgg acccagttct cactcccatt gggtgtcggg tttccagaga 60
   agccaatcag tgtcgtcgcg gtcgctgttc taaagcccgc acgcacccac cgggactcag 120
<210> 13
   <211> 112
   <212> DNA
   <213> Artificial Sequence
<220>
   <223> A-I1-1.1 HLA-A Intron 1 capture probe sequence
<400> 13
   gggaaacggc ctctgcgggg agaagcaagg ggcccgcccg gcgggggcgc aggacccggg 60
   aagccgcgcc gggaggaggg tcgggcgggt ctcagccact cctcgccccc ag 112
<210> 14
   <211> 112
   <212> DNA
   <213> Artificial Sequence
<220>
   <223> A-I1-1-2 HLA-A Intron 1 capture probe sequence
<400> 14
   gggaaaccgc ctctgcgggg agaagcaagg ggccctcctg gcgggggcgc aggaccgggg 60
   gagccgcgcc gggaggaggg tcgggcaggt ctcagccact gctcgccccc ag 112
<210> 15
   <211> 115
   <212> DNA
   <213> Artificial Sequence
<220>
   <223> A-I2-1.1 HLA-A Intron 2 capture probe sequence
<400> 15
   gcaggtcacg acccctcatc ccccacggac gggccaggtc gcccacagtc tccgggtccg        60
   agatccaccc cgaagccgcg gggctccgag acccttgtcc cgggagaggc ccagg            115
<210> 16
   <211> 111
   <212> DNA
   <213> Artificial Sequence
<220>
   <223> A-I2-2.1 HLA-A Intron 2 capture probe sequence
<400> 16
   gaggcccagg cgcctttaac ccggtttcat tttcagttta ggccaaaaat ccccccgggt        60
   tggtcggggc ggggcggggc tcgggggacc gggctgacct cggggtccgg g                111
<210> 17
   <211> 110
   <212> DNA
   <213> Artificial Sequence
<220>
   <223> A-I2-2.2 HLA-A Intron 2 capture probe sequence
<400> 17
   gaggcccagg cgcctttacc cggtttcatt ttcagtttag gccaaaaatc cccccgggtt        60
   ggtcggggcc gggcggggct cgggggactg ggctgaccgc ggggtcgggg                  110
<210> 18
   <211> 113
   <212> DNA
   <213> Artificial Sequence
<220>
   <223> A-I3-1.1 HLA-A Intron 3 capture probe sequence
<400> 18
   gatcgcctgt agatctccgg ggctggcctc ccacaagaaa gggagacaaa tgggaccaac        60
   actataatat cgccctccct ctggtcttga gggagaagaa tcctcctggg ttt              113
<210> 19
   <211> 113
   <212> DNA
   <213> Artificial Sequence
<220>
   <223> A-I3-1.2 HLA-A Intron 3 capture probe sequence
<400> 19
   gatcgcctgt agatctcccg ggctggcctc ccacaaggag gggagacaat tgggaccaac       60
   actagaatat cgccctccct ctggtcctga gggagaggaa tcctcctggg ttt             113
<210> 20
   <211> 103
   <212> DNA
   <213> Artificial Sequence
<220>
   <223> A-I3-2.1 HLA-A Intron 3 capture probe sequence
<400> 20
   ctgggttctg tgctctcttc cccatcccgg gtgtcctgtc cattctcaag atggccacat       60
   gcgtgctggt ggagtgtccc atgacagatg caaaatgcct gaa                        103
<210> 21
   <211> 103
   <212> DNA
   <213> Artificial Sequence
<220>
   <223> A-I3-2.2 HLA-A Intron 3 capture probe sequence
<400> 21
   ctgggttctg tgctctcttc cccatcccag gtgtcctgtc cattctcaag atagccacat       60
   gtgtgctgga ggagtgtccc atgacagatg caaaatgcct gaa                        103
<210> 22
   <211> 100
   <212> DNA
   <213> Artificial Sequence
<220>
   <223> A-I3-2.3 HLA-A Intron 3 capture probe sequence
<400> 22
   ctgggttctg tgctctcttc cccatcccgg gtgtcctgtc cattctcaag atggccacat       60
   gcatgctgga gtgtcccatg acagatgcaa aatgcctgaa                           100
<210> 23
   <211> 103
   <212> DNA
   <213> Artificial Sequence
<220>
   <223> A-I3-2.4 HLA-A Intron 3 capture probe sequence
<400> 23
   ctgggttctg tgctctcttc cccatcccag gtgtcctgtc catcctcaaa atggccacat 60
   gcgtgctggt ggagtgtccc atgacagatg caaaatggct gaa 103
<210> 24
   <211> 101
   <212> DNA
   <213> Artificial Sequence
<220>
   <223> A-I4-1.1 HLA-A Intron 4 capture probe sequence
<400> 24
   taaggaggga gatgggggtg tcatgtctct tagggaaagc aggagcctct ctggagacct 60
   ttagcagggt cagggcccct caccttcccc tcttttccca g 101
<210> 25
   <211> 98
   <212> DNA
   <213> Artificial Sequence
<220>
   <223> A-I4-1.2 HLA-A Intron 4 capture probe sequence
<400> 25
   taaggaggga gacgggggtg tcatgtcttt tagggaaagc aggagcctct ctgaccttta 60
   gcagggtcag ggcccctcac cttcccctct tttcccag 98
<210> 26
   <211> 96
   <212> DNA
   <213> Artificial Sequence
<220>
   <223> A-I4-1.3 HLA-A Intron 4 capture probe sequence
<400> 26
   taaggaggga gatgggggtg tcatgtcttt tacggaaagc aggagcctct ctgaccttta 60
   gcagggtcag ggcccctcac cttccccttt tcccag 96
<210> 27
   <211> 113
   <212> DNA
   <213> Artificial Sequence
<220>
   <223> A-I5-1.1 HLA-A Intron 5 capture probe sequence
<400> 27
   tctgagattt cttgtctcac tgagggttcc aagacccagg tagaagtgtg ccctgcctcg        60
   ttactgggaa gcaccatcca caattatggg cctacccagc ctgggccctg tgt              113
<210> 28
   <211> 113
   <212> DNA
   <213> Artificial Sequence
<220>
   <223> A-I5-1.2 HLA-A Intron 5 capture probe sequence
<400> 28
   tctgagattt cttgtctcac tgagggttcc aagccccagc tagaaatgtg ccctgtctca        60
   ttactgggaa gcaccatcca caatcatggg cctacccagc ctgggccctg tgt              113
<210> 29
   <211> 114
   <212> DNA
   <213> Artificial Sequence
<220>
   <223> A-I5-2.1 HLA-A Intron 5 capture probe sequence
<400> 29
   ccctgggtct gcagtcacac atttctggaa acttctctga ggtccaagac ttggaggttc        60
   ctctaggacc ttaaggccct gactcctttc tggtatctca caggacattt tctt             114
<210> 30
   <211> 114
   <212> DNA
   <213> Artificial Sequence
<220>
   <223> A-I5-2.2 HLA-A Intron 5 capture probe sequence
<400> 30
   ccctgggtct gcattcacac atttctggaa acttctctgg ggtccaaggc tacgaggttc        60
   ctctaggacc ttaaggccct ggctcctttc tggtagctca caggacattt tctt 114
<210> 31
   <211> 120
   <212> DNA
   <213> Artificial Sequence
<220>
   <223> B-3-1.1 HLA-B 3' untranslated region capture probe sequence
<400> 31
   cagaatttgt tcatgactgt tgttttctgt agcctgagac agctgtcttg tgagggactg 60
   agatgcagga tttcttcact cctccccttt gtgacttcaa gagcctctgg catctctttc 120
<210> 32
   <211> 114
   <212> DNA
   <213> Artificial Sequence
<220>
   <223> B-5-1.1 HLA-B 5' untranslated region capture probe sequence
<400> 32
   gatcaggacg aagtcccagg tcccggacgg ggctctcagg gtctcaggct ccgagggccg 60
   cgtctgcaat ggggaggcgc agcgttgggg attccccact cccatgagtt tcac 114
<210> 33
   <211> 114
   <212> DNA
   <213> Artificial Sequence
<220>
   <223> B-5-1.2 HLA-B 5' untranslated region capture probe sequence
<400> 33
   gatcaggacg aagtcccagg ccccgggcgg ggctctcagg gtctcaggct ccgagagccg 60
   cgtctgcatt ggggaggcgc agcgttgggg attccccact cccacgagtt tcac 114
<210> 34
   <211> 110
   <212> DNA
   <213> Artificial Sequence
<220>
   <223> B-5-2.1 HLA-B 5' untranslated region capture probe sequence
<400> 34
   ttcccactcc cattgggtgt cgggtgtcta gagaagccaa tcagtgtcgc cggggtccca 60
   gttctaaagt ccccacgcac ccacccggac tcaaaatctc ctcagacgcc 110
<210> 35
   <211> 110
   <212> DNA
   <213> Artificial Sequence
<220>
   <223> B-5-2.2 HLA-B 5' untranslated region capture probe sequence
<400> 35
   ttcccactcc cattgggtat tggatatcta gagaagccaa tcagcgtcgc cgcggtccca 60
   gttctaaagt ccccacgcac ccacccggac tcagagtctc ctcagacgcc 110
<210> 36
   <211> 110
   <212> DNA
   <213> Artificial Sequence
<220>
   <223> B-5-2.3 HLA-B 5' untranslated region capture probe sequence
<400> 36
   ttcccactcc cattgggtgt cggatatcta gagaagccaa tcagcgtcgc cggggtccca 60
   gttctaaagt ccccacgcac ccacccggac tcagagtctc ctcagacgcc 110
<210> 37
   <211> 101
   <212> DNA
   <213> Artificial Sequence
<220>
   <223> B-I1-1.1 HLA-B Intron 1 capture probe sequence
<400> 37
   gggaaatggc ctctgtgggg aggagcgagg ggaccgcagg cgggggcgca ggacccgggg 60
   agccgcgccg ggaggagggt cgggcccctc ctcgccccca g 101
<210> 38
   <211> 108
   <212> DNA
   <213> Artificial Sequence
<220>
   <223> B-I1-1.2 HLA-B Intron 1 capture probe sequence
<400> 38
   gggaaatggc ctctgccggg aggagcgagg ggaccgcagg cgggggcgca ggacctgagg 60
   agccgcgccg ggaggagggt cgggcgggtc tcagcccctc ctcacccc 108
<210> 39
   <211> 115
   <212> DNA
   <213> Artificial Sequence
<220>
   <223> B-I2-1.1 HLA-B Intron 2 capture probe sequence
<400> 39
   gtgagtgacc ccggcccggg gcgcaggtca cgactcccca tcccccacgg acggcccggg 60
   tcgccccgag tctccgggtc cgagatccgc ctccctgagg ccgcgggacc cgccc 115
<210> 40
   <211> 113
   <212> DNA
   <213> Artificial Sequence
<220>
   <223> B-I2-1.2 HLA-B Intron 2 capture probe sequence
<400> 40
   gtgagtgacc ccggcccggg gcgcaggtca cgactcccca tcccccacgt acggcccggg 60
   tcgccccgag tctccgggtc cgagatccgc ccccgaggcc gcgggacccg ccc 113
<210> 41
   <211> 115
   <212> DNA
   <213> Artificial Sequence
<220>
   <223> B-I2-1.3 HLA-B Intron 2 capture probe sequence
<400> 41
   gtgagtgacc ccggcctggg gcgcaggtca cgacccctcc ccaaccccga cgtacggccc 60
   gggtctcctc gagtctctag gtccgagatc cgccccgagg ccgcgggacc cgccc 115
<210> 42
   <211> 110
   <212> DNA
   <213> Artificial Sequence
<220>
   <223> B-I2-2.1 HLA-B Intron 2 capture probe sequence
<400> 42
   gccgcgggac ccgcccagac cctcgaccgg cgagagcccc aggcgcgttt acccggtttc    60
   attttcagtt gaggccaaaa tccccgcggg ttggtcgggg gggggcgggg              110
<210> 43
   <211> 110
   <212> DNA
   <213> Artificial Sequence
<220>
   <223> B-I2-2.2 HLA-B Intron 2 capture probe sequence
<400> 43
   gccgcgggac ccgcccagaa cctcgaccgc agagagcccc aggcgacttt acccggtttc    60
   attttcagtt gaggtcaaaa gccccgcggg ttggtcaggg gggggcgggg              110
<210> 44
   <211> 98
   <212> DNA
   <213> Artificial Sequence
<220>
   <223> B-I3-1.1 HLA-B Intron 3 capture probe sequence
<400> 44
   gtaccagggg cagtggggag ccttccccat ctcctatagg tcgccgggga tggcctccca    60
   cgagaagaag aggaaaatgg gatcagcgct agaatgtc                            98
<210> 45
   <211> 98
   <212> DNA
   <213> Artificial Sequence
<220>
   <223> B-I3-1.2 HLA-B Intron 3 capture probe sequence
<400> 45
   gtaccagggg cagtggggag ccttccccat ctcctataga tcgcccggga tggcctccca    60
   cgaggagggg aggaaaatga gagaagcgct agaatgtc                            98
<210> 46
   <211> 126
   <212> DNA
   <213> Artificial Sequence
<220>
   <223> B-I3-2.1 HLA-B Intron 3 capture probe sequence
<400> 46
   tgtctgggtt ctgtgcccct tccccacccc aggtgtcctg tccattctca ggctggtcac      60
   atgggtggtc ctagggtgtc ccatgaaaga tgcaaagcgc ctgaattttc tgactcttcc     120
   catcag                                                                126
<210> 47
   <211> 93
   <212> DNA
   <213> Artificial Sequence
<220>
   <223> B-I4-1.1 HLA-B Intron 4 capture probe sequence
<400> 47
   gtaaggaggg ggatgagggg tcatatctct tctcagggaa agcaggagcc cttcagcagg      60
   gtcagggccc ctcatcttcc cctcctttcc cag                                   93
<210> 48
   <211> 104
   <212> DNA
   <213> Artificial Sequence
<220>
   <223> B-I4-1.2 HLA-B Intron 4 capture probe sequence
<400> 48
   gtaaggaggg ggatgagggg tcatatctct tctcagggaa agcaggagcc cttctggagc      60
   ccttcagcag ggtcagggcc cctcatcttc ccctcctttc ccag                      104
<210> 49
   <211> 115
   <212> DNA
   <213> Artificial Sequence
<220>
   <223> B-I5-1.1 HLA-B Intron 5 capture probe sequence
<400> 49
   gtagggaagg ggtgaggggt ggggtctggg ttttcttgtc ccactggggg tttcaagccc      60
   caggtagaag tgttccctgc ctcattactg ggaagcagca tgcacacagg ggcta          115
<210> 50
   <211> 120
   <212> DNA
   <213> Artificial Sequence
<220>
   <223> B-I5-2.1 HLA-B Intron 5 capture probe sequence
<400> 50
   ctttcctcgt gtttcctgat cctgccctgg gtctgtagtc atacttctgg aaattctttt      60
   tgggtccaag actaggaggt tcctctaaga tctcatggcc ctgcttcctc ccagtcccct     120
<210> 51
   <211> 115
   <212> DNA
   <213> Artificial Sequence
<220>
   <223> C-3-2.1 HLA-C 3' untranslated region capture probe sequence
<400> 51
   gacagctgcc tgtgtgggac tgagatgcag gatttcttca cacctctcct ttgtgacttc      60
   aagagcctct ggcatctctt tctgcaaagg catctgaatg tgtctgcgtt cctgt          115
<210> 52
   <211> 110
   <212> DNA
   <213> Artificial Sequence
<220>
   <223> C-5-1.1 HLA-C 5' untranslated region capture probe sequence
<400> 52
   caggacgaag tcccaggtcc cgggcggggc tctcagggtc tcaggctcca agggccgtgt      60
   ctgcactggg gaggcgccgc gttggggatt ctccactccc ctgagtttca                110
<210> 53
   <211> 116
   <212> DNA
   <213> Artificial Sequence
<220>
   <223> C-5-1.2 HLA-C 5' untranslated region capture probe sequence
<400> 53
   caggacgaag tcccaggtcc cgggcggggc tctctgggtc tcaagctccc agggccgtgt      60
   ctgcattggg gaggcgcagc gttggggatt ccccactccc actcccctga gtttca         116
<210> 54
   <211> 109
   <212> DNA
   <213> Artificial Sequence
<220>
   <223> C-5-2.1 HLA-C 5' untranslated region capture probe sequence
<400> 54
   ctcatgacgc gtccccaatt cccactccca ttgggtgtcg gattctagag aagccaatca 60
   gcgtctccgc agtcccggtt ctgaagtccc cagtcaccca cccggactc 109
<210> 55
   <211> 102
   <212> DNA
   <213> Artificial Sequence
<220>
   <223> C-5-2.2 HLA-C 5' untranslated region capture probe sequence
<400> 55
   ctcatgacgc gtccccaatt cccactccca ttgggtgtcg ggttctagag aagccaatca 60
   gcgtctacgc agtcccggtt ctgaagtcac ccacccggac tc 102
<210> 56
   <211> 112
   <212> DNA
   <213> Artificial Sequence
<220>
   <223> C-I1-1.1 HLA-C Intron 1 capture probe sequence
<400> 56
   ggttgggagg gaagcggcct ctgcggagag gagcgagggg cccgcccggc gagggcgcag 60
   gacccgggga gccgcgcagg gaggtgggtc gggcgggtct cagcccctcc tc 112
<210> 57
   <211> 97
   <212> DNA
   <213> Artificial Sequence
<220>
   <223> C-I2-1.1 HLA-C Intron 1 capture probe sequence
<400> 57
   gtgagtgacc ccggcccggg gcgcaggtca cgacccctcc ccatccccca cggacggccc 60
   gggtcgcccc gagtctcccg gtctgagatc caccccg 97
<210> 58
   <211> 97
   <212> DNA
   <213> Artificial Sequence
<220>
   <223> C-I2-1.2 HLA-C Intron 1 capture probe sequence
<400> 58
   gtgagtgacc ccggcccggg gcgcaggtca cgacccctcc ccatccccca cggacggccc 60
   gggtcgccta gagtctcccc gtctgagatc cacccca 97
<210> 59
   <211> 114
   <212> DNA
   <213> Artificial Sequence
<220>
   <223> C-I2-2.1 HLA-C Intron 2 capture probe sequence
<400> 59
   gcggaacccg cccagaccct cgaccggaga gagccccagt cacctttacc cggtttcatt 60
   ttcagtttag gccaaaatcc ccgcgggttg gtcggggcgg ggcggggctc gggg 114
<210> 60
   <211> 115
   <212> DNA
   <213> Artificial Sequence
<220>
   <223> C-I2-2.2 HLA-C Intron 2 capture probe sequence
<400> 60
   gcggaacccg cccagaccct cgaccggaga gagccctagt cgcctttacc cggtttcatt 60
   ttcagtttag gccaaaatcc ccgcgggttg gtcggggctg gggcggggtt cgggg 115
<210> 61
   <211> 115
   <212> DNA
   <213> Artificial Sequence
<220>
   <223> C-I2-2.3 HLA-C Intron 2 capture probe sequence
<400> 61
   gcggaacccg cccagaccct cgaccggaga gagccccagt cacctttacc cggtttcatt 60
   ttcagtttag gccaaaatcc ccgcgggttg gtcggggctg gggcggggct cgggg 115
<210> 62
   <211> 108
   <212> DNA
   <213> Artificial Sequence
<220>
   <223> C-13-1.1a HLA-C Intron 3 capture probe sequence
<400> 62
   gggcagtggg gagccttccc catctcctgt agatctcccg ggatggcctc ccacgaggag 60
   gggaggaaaa tgggatcagc gctagaatat cgccctccct tgaatgga 108
<210> 63
   <211> 110
   <212> DNA
   <213> Artificial Sequence
<220>
   <223> C-I3-1.1b HLA-C Intron 3 capture probe sequence
<400> 63
   cccccttccc caccccaggt gtcctgtcca ttctcaggat agtcacatgg gcgctgttgg 60
   agtgtcgcaa gagagataca aagtgtctga attttctgac tcttcccgtc 110
<210> 64
   <211> 110
   <212> DNA
   <213> Artificial Sequence
<220>
   <223> C-I3-1.2 HLA-C Intron 3 capture probe sequence
<400> 64
   ccgccttccc caccccaggt gtcctgtcca ttctcaggat ggtcacatgg gcgctgctgg 60
   agtgtcccaa gagagatgca aagtgtctga attttctgac tcttcccgtc 110
<210> 65
   <211> 117
   <212> DNA
   <213> Artificial Sequence
<220>
   <223> C-I4-1.1 HLA-C Intron 4 capture probe sequence
<400> 65
   ggagggggat gaggggtcat gtgtcttctc agggaaagca gaagtcctgg agcccttcag        60
   ccgggtcagg gctgaggctt gggggtcagg gcccctcacc ttcccctcct ttcccag          117
<210> 66
   <211> 100
   <212> DNA
   <213> Artificial Sequence
<220>
   <223> C-I4-1.2 HLA-C Intron 4 capture probe sequence
<400> 66
   ggagggggat gaggggtgat atctgttctc agggaaagca ggagcccttc tggagccctt        60
   cagcagggtc agggcccctc accttcccct cctttcccag                             100
<210> 67
   <211> 120
   <212> DNA
   <213> Artificial Sequence
<220>
   <223> C-I4-1.3 HLA-C Intron 4 capture probe sequence
<400> 67
   ggaggggaat ggggggtcac atctcttatc agagaaagca gaagtccttc tggagccctt         60
   cagccgggtc agggctgagg cttgggggtc agggcccctc accttctcct cctttcccag       120
<210> 68
   <211> 120
   <212> DNA
   <213> Artificial Sequence
<220>
   <223> C-I4-1.4 HLA-C Intron 4 capture probe sequence
<400> 68
   ggagggggat gaggggtcat gtgtcttctc agggaaagca gaagtccttc tggagccctt        60
   cagccgggtc agggctgagg cttgggtgta agggcccctc accttcccct cctttcccag       120
<210> 69
   <211> 110
   <212> DNA
   <213> Artificial Sequence
<220>
   <223> C-I5-1.1 HLA-C Intron 5 capture probe sequence
<400> 69
   gggtctgggt tttcttgtcc cactgggagt ttcaagcccc aggtagaagt gtgccccacc 60
   tcgttactgg aagcaccatc cacacctggg ccatcccagc ctgggaccct 110
<210> 70
   <211> 118
   <212> DNA
   <213> Artificial Sequence
<220>
   <223> C-I5-2-1 HLA-C Intron 5 capture probe sequence
<400> 70
   cctgggtctg cagtcatagt tctggaaact tctcttgggt ccaagactag gaggttcccc 60
   taagatcgca tggccctgcc tcctccctgt cccctcacag ggcattttct tcccacag 118
<210> 71
   <211> 118
   <212> DNA
   <213> Artificial Sequence
<220>
   <223> C-I5-2-2 HLA-C Intron 5 capture probe sequence
<400> 71
   cctgggtctg cagtcgtagt tctggaaact tctcttgggt ccaagactag gaggttcccc 60
   taaaatcaca tggccctgcc tcctcccagt cccctcatag ggcattttct tcccacag 118

## Description

### FIELD

The present invention relates to a method of genotyping highly polymorphic nucleic acid. In particular, the present disclosure relates to methods for genotyping highly polymorphic HLA gene alleles using high-throughput sequencing technology.

### BACKGROUND

The so called next generation sequencing (NGS) technology has accelerated genetic research and enabled previously undiagnosed genetic defects to be rapidly characterized, resulting in early disease diagnosis and improved health outcomes. A key feature of next generation sequencing technology is the ability to sequence millions of single DNA molecules at the same time. The parallelism and speed of sequencing is such that it is now possible to sequence an entire human genome in just a few days. In addition to being able to sequence entire complex genomes by NGS many applications are also focusing on the clinical potential of NGS and the detection of genetic variants in specific, targeted regions.

One method of targeted genetic sequencing involves the use of labelled probes that bind to target regions which can then be extracted from genomic DNA.

The probes used can be RNA or DNA in nature and are usually in the order of 100 nucleotides in length.

The utilization of capture probes is useful for genetic regions, which in healthy individuals, tend not to be very polymorphic. That is, the genetic sequence differences between healthy individuals are in the order of <1%. A high redundancy of over lapping probes are used to ensure that if there are sequence differences between the probes and the target DNA that prevent the probe from annealing to the target sequence there will be other probes adjacent to the genetic differences that enable the extraction and sequencing of the variant DNA fragments.

Whilst the use of capture probes has wide applications, their use is not universal and considered inappropriate for the genotyping of highly polymorphic genes, like the genes of the HLA system. HLA is central to our immune response to pathogens. HLA molecules reside on the surface of cells and present fragmented pathogen peptides to the immune system. Most individuals have unique HLA types enabling the species to be able to defend against practically any pathogenic attack.

Whilst the advantages of HLA polymorphism to our species is obvious, the flipside is that HLA antigens on transplanted organs, including hematopoietic stem cells (HSC), are seen by the recipient's immune system as "foreign" resulting in organ rejection. In the case of HSC transplants, the transplanted stem cells target the new host resulting in graft versus host disease (GvHD), a potentially fatal consequence of HSC transplants.

Therefore, genotyping of HLA is required to ensure compatibility between donors of HSC and patients. There are several HLA genes, all of which reside within approximately 4 million bases in a region known as the Major Histocompatibility Complex (MHC) located on the short arm of chromosome 6 in humans. The genes can be divided into 2 sets. The Class I genes which include HLA-A, HLA-B and HLA-C, and the class 2 genes that include HLA-DRB1, HLA-DQB1 and HLA-DPB1. It is these genes that are usually typed for transplantation purposes, but the MHC also contains additional class I HLA genes that are not very polymorphic and have functions other than pathogen derived peptide presentation. These genes include HLA-E, -F and -G. Furthermore there are numerous class I and class 2 like pseudogenes and gene fragments.

The classical class I genes are similar in sequence. They are all relatively small genes of approximately 3,200 base pairs characterized by 7-8 exons and small introns. The class 2 genes have larger introns, and some are in the order of 20,000 base pairs.

The high level of HLA polymorphism is generated by the "sharing" of sequence motifs from alleles of the same and different loci. There are relatively few unique polymorphisms and SNPs. In other words, newly identified alleles will contain a unique combination of motifs that are seen in other alleles of the same locus or from alleles of other, related, loci. Motif sharing is an effective mechanism for the rapid evolution of polymorphism and serves the species well when encountering pathogens. The size of the motif shared between alleles and loci varies. In some cases the motif may be less than 20 bp, but other alleles may contain half the entire gene. The consequence of the high level of polymorphism is that in some cases it is impossible to identify HLA matched HSC donors.

NGS for HLA offers significant benefits over conventional Sanger based sequencing techniques. Conventional HLA sequencing techniques require the sequencing of DNA amplified by polymerase chain reaction. The amplified DNA is sequenced as a plurality of sequence molecules where it cannot be determined if adjacent nucleotides are from the same or different (maternal or paternal) chromosomes. This inability to phase sequence frequently results in an inaccurate typing and a subsequent experiment is required to resolve the ambiguous type.

NGS produces single molecule sequence data, complete with phase information that can be compiled with sequence analysis software, such as Assign MPS to potentially produce an unambiguous HLA type. However, when sequencing PCR products, PCR incorporated errors and chimeric molecules are also sequenced, creating sequence analysis difficulties.

However, conventional exon based probe capture techniques are likely to be unreliable as HLA types are largely generated by motif shuffling and the degree of sequence difference can be substantial from one allele to the next.

Thus, there remains a need for a method that enables high-throughput sequencing of highly polymorphic genes, such as the HLA genes.

WO 2010/151416 describes consensus sequences of introns from HLA-A, HLA-B, and HLA-C allotypes used to develop primers for use as sequencing primers to determine the HLA alleles in sequence-based HLA typing.

US 2004/0197775 describes a method for detection of at least one allele of a genetic locus which can be used to provide direct determination of the haplotype. The method comprises amplifying genomic DNA with a primer pair that spans an intron sequence and defines a DNA sequence in genetic linkage with an allele to be detected.

Gabriel C. et al., Rapid high-throughput human leukocyte antigen typing by massively parallel pyrosequencing for high-resolution allele identification. Human Immunology 70(11) 960-964. DOI: 10.1016/J.HUMIMM.2009.08.009, describes a HLA sequence-based typing method using the Roche/454 Life Sciences Genome Sequencer FLX system.

### SUMMARY

The present inventors describe a capture probe method that is suitable for identifying alleles in highly polymorphic HLA genes by targeting capture probes to non-coding sequences. The non-coding regions of commonly typed HLA class I and class II molecules are less polymorphic than the polymorphic exons and the number of possible alternative sequences in different HLA types would appear to be relatively finite except for random SNPs that would occur through the normal process of variant generation. Thus, a small number of probes can be designed at single region that will enable the capture of all alleles, including those likely to be described in the future. The capture probes will capture DNA fragments that include the polymorphic exons and the sequencing of the capture DNA fragment will produce sequence of the polymorphic fragments enabling the deduction of an HLA type.

Accordingly, the invention provides a method for identifying one or more HLA gene alleles in a subject, the method comprising:
a) contacting a nucleic acid sample from the subject with oligonucleotide probes, wherein the oligonucleotide probes hybridize to HLA gene target sequences in the nucleic acid sample;
b) enriching for a nucleic acid that is hybridized to an oligonucleotide probe by separating it from nucleic acid not bound to an oligonucleotide probe; and
c) sequencing the enriched nucleic acid by high-throughput screening to identify one or more HLA gene alleles;
wherein the gene target sequences are in a non-coding region of one or more HLA genes, and wherein the oligonucleotide probes are suitable for preparing the nucleic acid for high-throughput screening using target capture.

The skilled person will understand that in preparing captured nucleic acid for sequencing, it may be desirable to amplify the captured nucleic acid. Accordingly, in one embodiment, the method comprises amplifying the nucleic bound to the oligonucleotide probe.

In one embodiment, the method comprises sequencing an HLA gene exon.

In another embodiment, the method comprises sequencing an entire HLA gene.

To facilitate enrichment of a nucleic acid of interest, in one embodiment the oligonucleotide probes comprise a capture tag.

In one embodiment, the capture tag is a hybridization tag.

In another embodiment, the capture tag is biotin or streptavidin.

In yet another embodiment, the method comprises contacting the capture tag with a binding agent.

In one embodiment, the binding agent is biotin or streptavidin.

In yet another embodiment, the binding agent is coupled to a substrate. In one particular embodiment, the binding agent is coupled to a magnetic substrate, such as for example a magnetic bead.

In one embodiment, the nucleic acid sample contacted with the oligonucleotide probes comprises single stranded nucleic acid.

In yet another embodiment, the nucleic acid sample is fragmented prior to being contacted with the oligonucleotide probes.

In one embodiment, the method comprises contacting nucleic acid fragments having an average length greater than about 100 bp with the oligonucleotide probes.

In another embodiment, the method comprises contacting nucleic acid fragments having an average length greater than about 2 kb with the oligonucleotide probes.

Alternatively, the nucleic acid sample is fragmented after it has been contacted with the oligonucleotide probes.

The oligonucleotide probes target non-coding regions of HLA genes, such as, for example, introns and intergenic regions. In one embodiment, the non-coding region of the HLA gene is an intron.

In another embodiment, the method further comprises detecting one or more alleles of a non-HLA gene.

In the method of the present invention, the sequencing is high-throughput sequencing.

In one embodiment, the high-throughput sequencing is a next-generation sequencing technique.

In a further embodiment, the present invention provides a method for identifying HLA gene alleles in a subject, the method comprising:
a) fragmenting the nucleic acid sample obtained from a subject to obtain nucleic acid fragments of at least about 2 kb in length;
b) contacting the single-stranded nucleic acid with oligonucleotide probes comprising a capture tag, wherein the oligonucleotide probes hybridize to HLA target sequences in the nucleic acid sample;
c) enriching for a nucleic acid that is hybridized to an oligonucleotide probe by contacting the capture tag with a binding agent; and
d) sequencing the nucleic acid by high-throughput sequencing to identify one or more HLA gene alleles;
wherein the HLA target sequences are in a non-coding region of one or more HLA genes, and wherein the oligonucleotide probes are suitable for preparing the nucleic acid for high-throughput screening using target capture.

While the nucleic acid used in the method of the invention may be from a pre-obtained sample, in one embodiment, the method comprises isolating nucleic acid from a biological sample obtained from the subject.

In one embodiment, the nucleic acid sample is fragmented before being contacted with the oligonucleotide probes.

In another embodiment, the nucleic acid sample is fragmented after being contacted with the oligonucleotide probes.

In one embodiment of the first or second aspect, one or more of the oligonucleotide probes comprises a nucleic acid sequence at least 95% identical to any one of SEQ ID Nos:1-8 or 10-71.

In another embodiment, one or more of the oligonucleotide probes comprises a nucleic acid sequence at least 98% identical to any one of SEQ ID Nos:1-8 or 10-71.

In one particular embodiment, one or more of the oligonucleotide probes comprises a nucleic acid sequence identical to any one of SEQ ID Nos:1-8 or 10-71.

In a third aspect, the present invention provides a method of identifying a transplant donor for a recipient in need of a transplant, the method comprising:
a) performing the method of the invention to identify one or more HLA gene alleles in the recipient in need of a transplant; and
b) identifying a transplant donor based on the presence of one or more alleles in an HLA gene of both the transplant donor and the transplant recipient.

The present invention further provides a method of reducing the likelihood of a transplant recipient developing graft versus host disease, the method comprising:
a) performing the method of the invention to identify one or more HLA gene alleles in the recipient in need of a transplant; and
b) identifying a transplant donor based on the presence of one or more HLA gene alleles in both the transplant donor and the transplant recipient;
wherein the presence of the one or more HLA gene alleles in both the transplant recipient and the transplant donor is indicative of a reduced likelihood of the transplant recipient developing graft versus host disease following transplantation of a graft from the transplant donor.

As will be apparent, preferred features and characteristics of one aspect of the invention are applicable to many other aspects of the invention.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The invention is hereinafter described by way of the following non-limiting Examples and with reference to the accompanying figures.

### BRIEF DESCRIPTION OF THE FIGURES

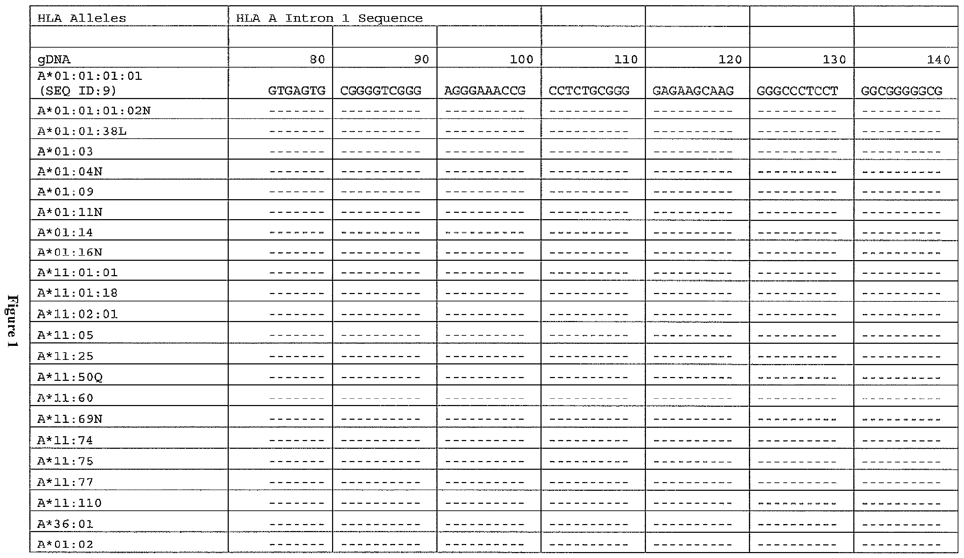

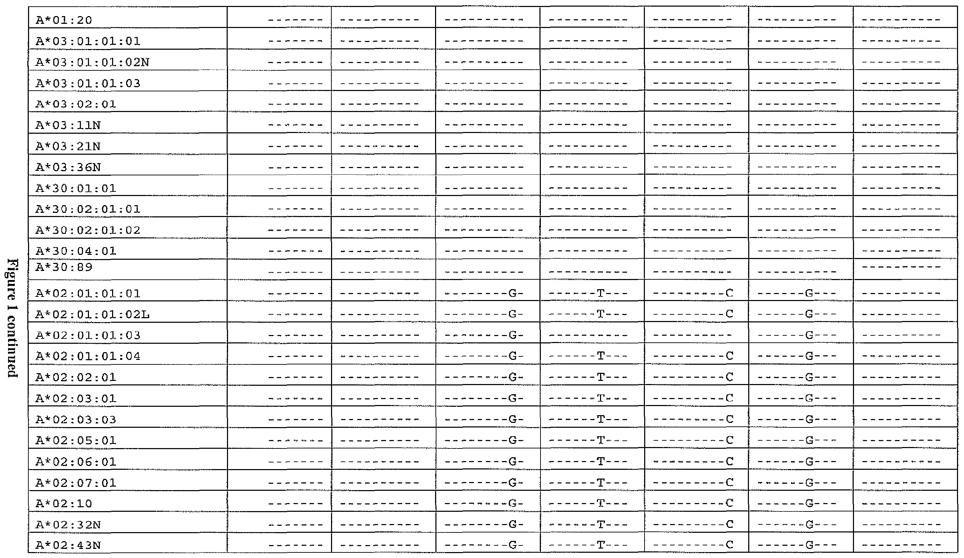

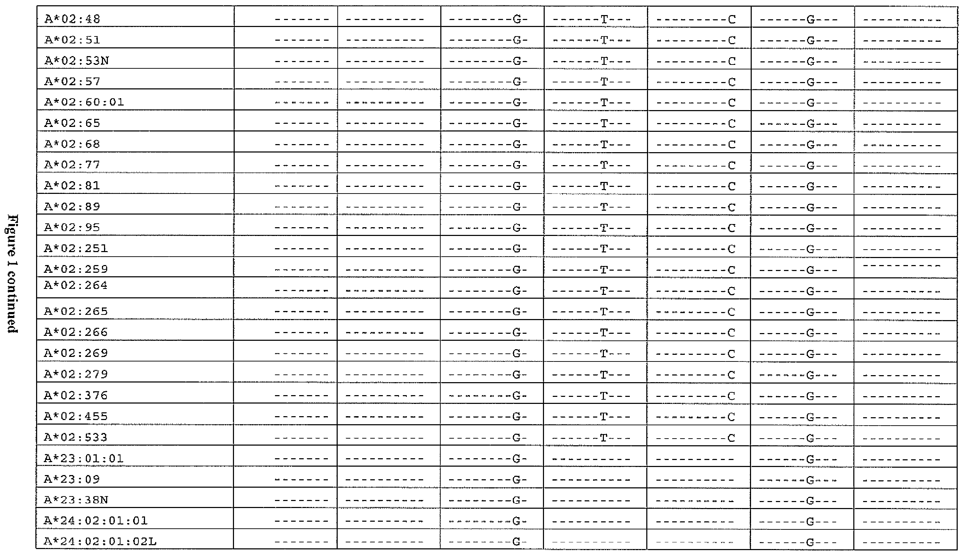

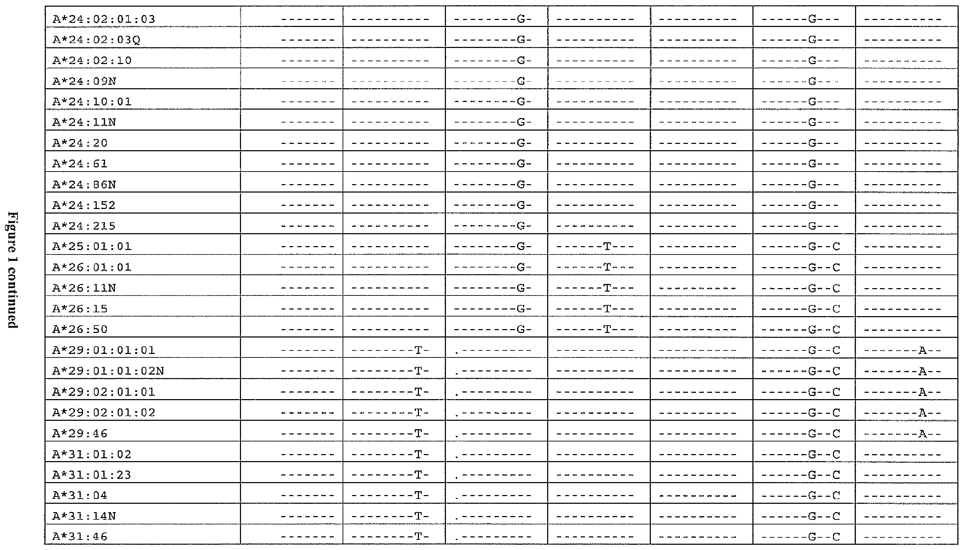

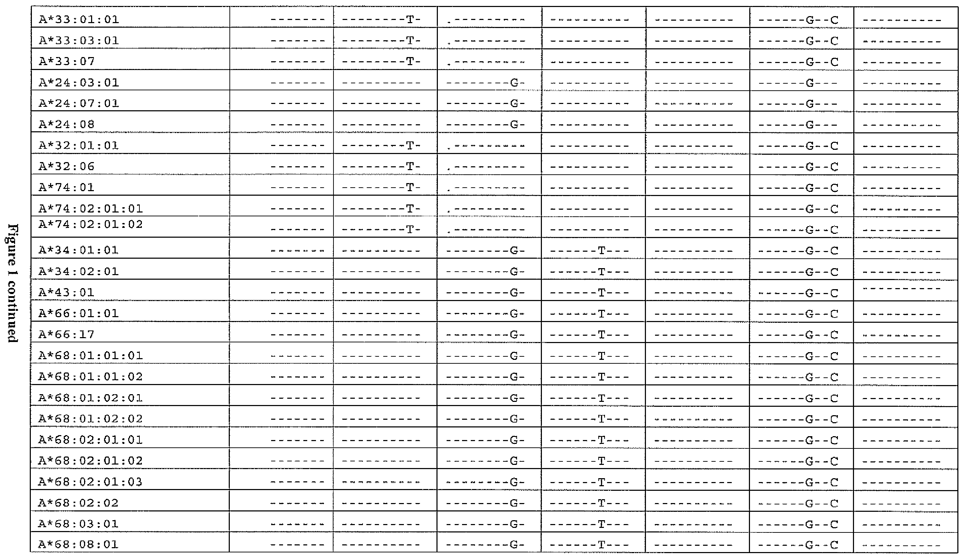

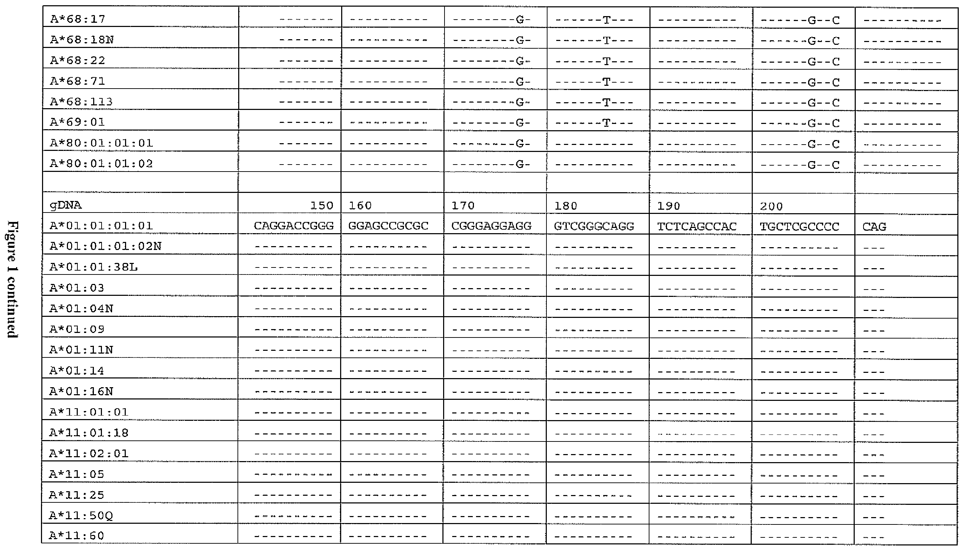

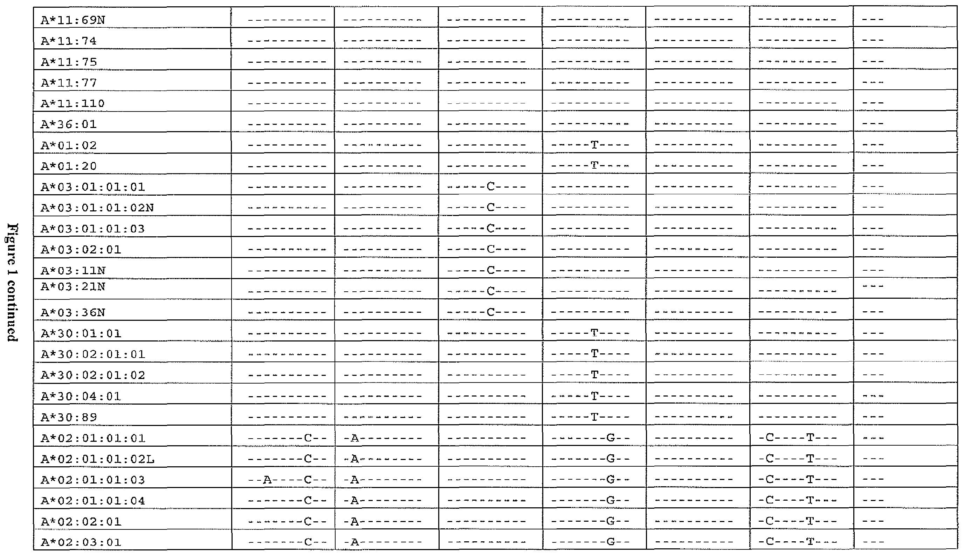

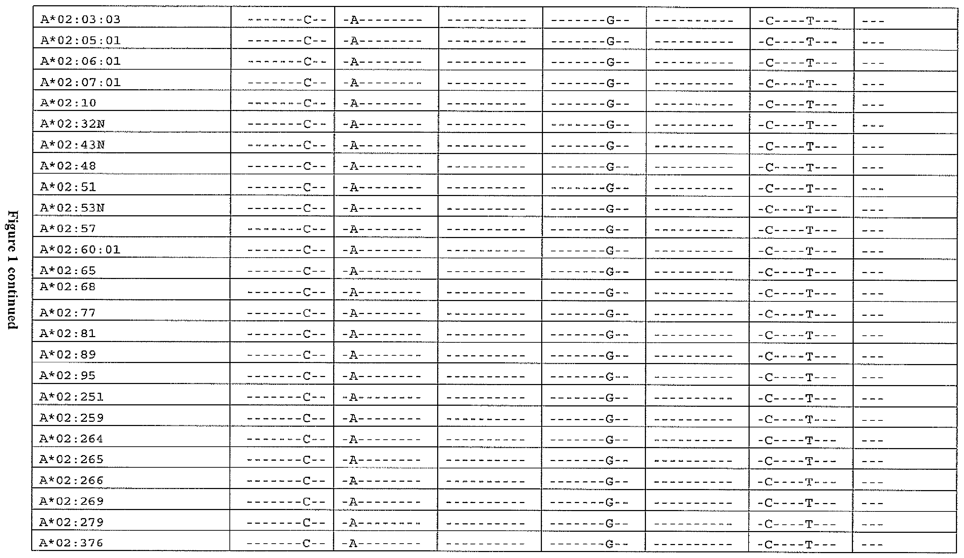

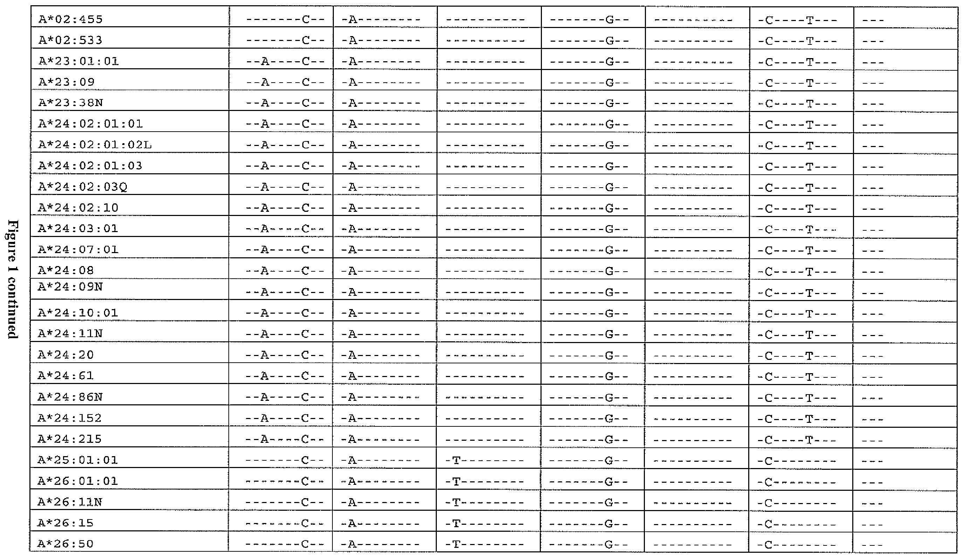

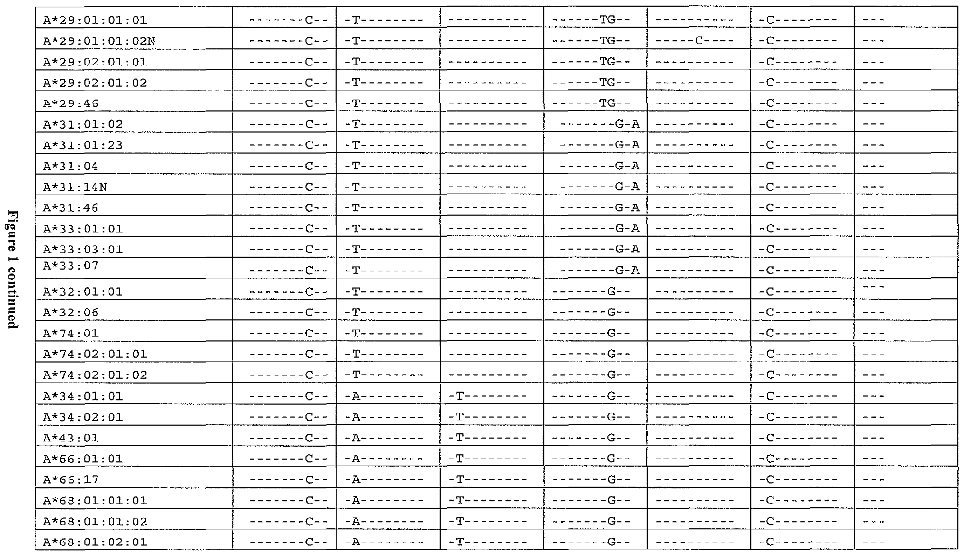

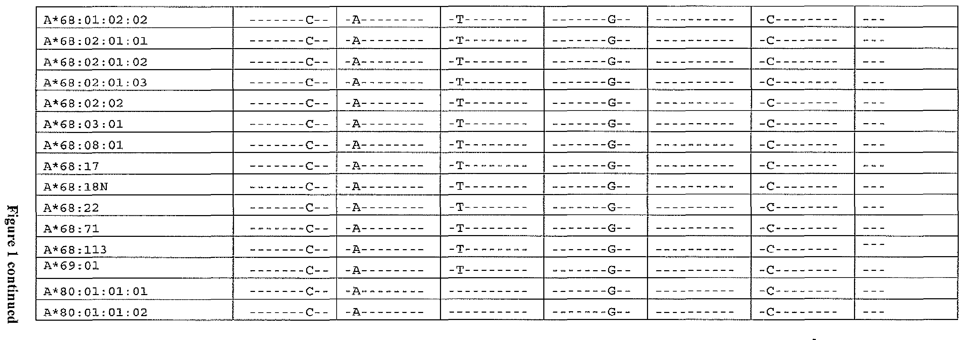

**Figure 1****.** Sequence alignment of HLA-A intron 1 demonstrating that different HLA alleles have identical and predictable HLA sequence. HLA allele nomenclature utilizes up to 4 fields separated by a colon (eg A^{∗}01:01:01:01). The first field represents, in general amino acid sequence differences that can be detected by antibodies. The second field represents nucleotide differences that result in amino acid changes but which may not be detectable with the use of antibodies. The third field represent synonymous changes and the forth field represents changes in non-coding regions. The use of letter code suffixes, such as the N in A^{∗}01:01:01:02N, indicates that the allele is an expression variant. For example N = Null or not expressed. As can be seen all A^{∗}01 alleles have identical sequence, except for 2 (A^{∗}01:02 and A^{∗}01:20) that share a nucleotide with A^{∗}30 alleles, and A^{∗}03 and A^{∗}11 alleles are also identical to the majority of A^{∗}01 alleles. Given that a probe will equally capture DNA that have a single base pair mismatch, a single probe should capture all A^{∗}01, A03, A^{∗}11 and A^{∗}30 alleles.
**Figure 2****.** The use of Capture probes to low polymorphic non-coding regions to capture and sequence polymorphic coding regions.
**Figure 3****.** HLA-A gene structure, showing the exons and location of the capture probes in the proof of principle experiments
**Figure 4****.** Analysis of sequence data generated using a targeted capture sequencing protocol (colors not shown).
   1: Shows the gene structure for HLA above a white to red shaded region that summarizes the sequence depth across the gene. White regions indicate sequence read depths of <50 and the closer the shading to red, the less sequence read depths. Blocks of black indicate no sequence files spanning this region.
   2: This shows the sample name (Sample 01) and the locus being analysed (HLA-A = gA)
   3: This region summarises the data. Each grey vertical bar represents the amount of sequence at that position. The maximum coverage shown by this scale is 100. The horizontal line of coloured boxes is the consensus sequence where red = T, black = G, blue = C and green = A. The coloured boxes underneath represent other sequences seen at that position and where they lie on the vertical grey bar indicates the percentage of reads with that base at that position. These sequences usually represent base call errors but can indicate heterozygous sequence. The black horizontal lines indicate the percentage of base calls, so a blue box on the first horizontal line indicates that 1% of base calls at that position are C. The scale is logarithmic so that the second horizontal line indicates 10% of reads,
   4: This region within the software interface indicates the best matched genotype between the consensus sequence and the HLA-A library. The first 2 columns indicate the genotypes. The 3rd column indicates the number of sequence mismatches with the coding sequences of HLA-A. The next column indicates the number of mismatches with the, non-coding sequences of HLA-A and the 5th and 6th columns indicate sequence mismatches when the sequence data is phased. The correct genotype will have 0 mismatches in columns 3 to 6. In this case the best matched genotype is with HLA-A^{∗}02:01:01:01+A^{∗}02:01:01:01.

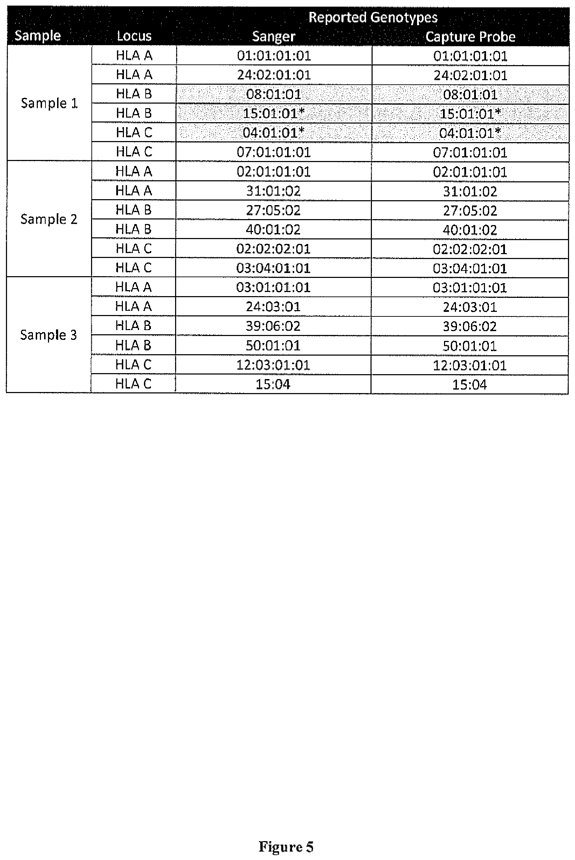

**Figure 5****.** Results of Next generation Sequencing following Capture Probe enrichment of 3 samples also sequenced by Sanger.

### KEY TO THE SEQUENCE LISTING

SEQ ID NOs:1 to 8 - Oligonucleotide capture probes
SEQ ID NO:9 - HLA A Intron 1
SEQ ID NOs:10 to 71 - HLA capture probe sequences

### DETAILED DESCRIPTION

### General Techniques and Definitions

Unless specifically defined otherwise, all technical and scientific terms used herein shall be taken to have the same meaning as commonly understood by one of ordinary skill in the art (e.g., in molecular genetics, biochemistry, and immunology).

Unless otherwise indicated, the molecular genetics, biochemistry, and immunological techniques utilized in the present invention are standard procedures, well known to those skilled in the art. Such techniques are described and explained throughout the literature in sources such as, J, Perbal, A Practical Guide to Molecular Cloning, John Wiley and Sons (1984), J. Sambrook and Russell., Molecular Cloning: A Laboratory Manual, 3rd edn, Cold Spring Harbour Laboratory Press (2001), R. Scopes, Protein Purification - Principals and Practice, 3rd edn, Springer (1994), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F.M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J.E. Coligan et al. (editors) Current Protocols in Immunology, John Wiley & Sons (including all updates until present).

The term "nucleic acid" or "nucleic acid sequence" or "nucleic acid molecule" refers to deoxyribonucleotides or ribonucleotides and polymers thereof in either single- or double-stranded form. The term nucleic acid is used interchangeably with gene, complementary DNA (cDNA), messenger RNA (mRNA), oligonucleotide, and polynucleotide.

By "isolated nucleic acid molecule" we mean a nucleic acid molecule which has generally been separated from the nucleotide sequences with which it is associated or linked in its native state (if it exists at all in nature). Preferably, the isolated nucleic acid is at least 60% free, more preferably at least 75% free, and more preferably at least 90% free from other components with which it is normally associated. The nucleic acid may be isolated from a biological sample using any suitable known technique. For example, total genomic DNA may be extracted from cells using methods known in the art and/or commercially available kits, e.g., by using the QIAamp DNA blood Mini Kit or the DNeasy Blood & Tissue Kit supplied by QIAGEN, or by using methods such as phenol/chloroform extraction and ethanol precipitation.

As used herein, the term "about", unless stated to the contrary, refers to +/-20%, more preferably +/-10%, or more preferably about +/-5% of the designated value.

### Method for Identifying HLA Gene Alleles

The present inventors have now developed a method of identifying HLA gene alleles that is particularly suited to targeted capture and high-throughput sequencing techniques. The method of the invention comprises contacting a nucleic acid sample from a subject with oligonucleotide probes in order to separate genomic regions of interest from the DNA sample. Whereas prior art methods of targeted capture have relied on oligonucleotides that capture coding regions of genes of interest, these methods are not suited to genotyping highly polymorphic genes due to the difficulty in designing exon-based capture probes that are able to identify all currently described alleles as well as identify new alleles. In contrast, the present invention utilizes oligonucleotide capture probes that hybridize to HLA gene target sequences that are located in non-coding regions, thus enabling high-throughput sequencing of highly polymorphic HLA gene alleles.

Figure 1 shows a sequence alignment of HLA-A intron 1 demonstrating that different HLA alleles have identical and predictable HLA intron sequence. As can be seen, all A^{∗}01 alleles have identical sequence, except for 2 (A^{∗}01:02 and A^{∗}01:20) that share a nucleotide with A^{∗}30 alleles, and A^{∗}03 and A^{∗}11 alleles are also identical to the majority of A^{∗}01 alleles. Given that a probe will equally capture DNA that have a single base pair mismatch, a single probe should capture all A^{∗}01, A03, A^{∗}11 and A^{∗}30 alleles.

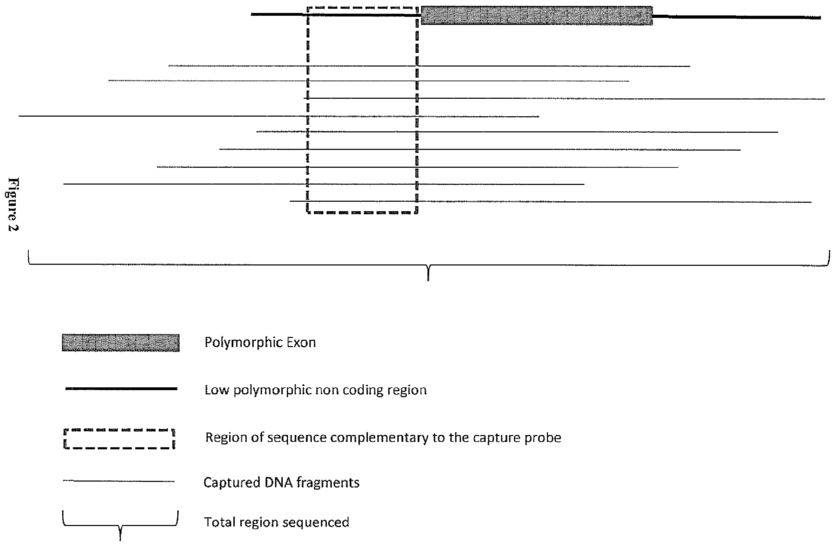

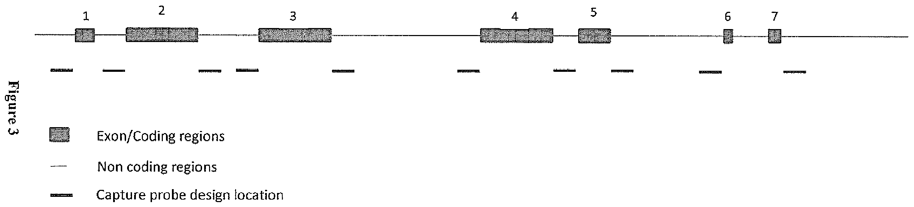

A diagram of showing the location in captured DNA fragments of sequence that is complementary to the capture probe, as well as its relative position to the polymorphic exon is provided in Figure 2. Figure 3 demonstrates how capture probes are designed and located in order to capture the polymorphic exons for sequencing.

As used herein, the terms "non-coding sequence" or "non-coding region" refer to nucleic acid sequences that are not part of the protein coding sequence of the gene in which alleles are identified. Thus, the terms include reference to introns, 5' and 3' un-translated regions, promoters, transcriptional regulatory elements and/or intergenic DNA. Thus, the non-coding region may be either internal to the gene of interest, or external to the gene of interest, for example, in an intergenic region. In one embodiment, the oligonucleotide probes hybridize to gene target sequences in introns in the gene of interest.

### Preparation of nucleic acid

The method of the invention may be performed on a nucleic acid sample that has already been obtained from a subject using any suitable technique known in the art. Alternatively, in one embodiment, the method comprises obtaining a nucleic acid sample from a biological sample that has been obtained from the subject. As used herein, a "biological sample" may be for instance lymphocytes, whole blood, buccal swab, biopsy sample or frozen tissue or any other sample comprising genomic DNA. Although almost any tissue source can be used for molecular genotyping, lymphocytes from peripheral blood, for example, are most often used. It is also possible to utilize samples obtained through non-invasive means, for example by way of cheek swab or saliva-based DNA collection. Various suitable methods for extracting DNA from such sources are known in the art. These range from organic solvent extraction to absorption onto silica coated beads and anion exchange columns. Automated systems for DNA extraction are also available commercially and may provide good quality, high purity DNA.

The nucleic acid used in the method of the invention may be single-stranded and/or double-stranded genomic DNA. In some embodiments, the nucleic acid may include long nucleic acids comprising a length of at least about 1 kb, at least about 2 kb, at least about 5 kb, at least about 10 kb, or at least about 20 kb or longer. Long nucleic acids can be prepared from sources by a variety of methods well known in the art. Methods for obtaining biological samples and subsequent nucleic acid isolation from such samples that maintain the integrity (i.e., minimize the breakage or shearing) of nucleic acid molecules are preferred. Exemplary methods include, but are not limited to, lysis methods without further purification (e.g., chemical or enzymatic lysis method using detergents, organic solvents, alkaline, and/or proteases), nuclei isolation with or without further nucleic acid purification, isolation methods using precipitation steps, nucleic acid isolation methods using solid matrices (e.g., silica-based membranes, beads, or modified surfaces that bind nucleic acid molecules), gel-like matrices (e.g., agarose) or viscous solutions, and methods that enrich nucleic acid molecules with a density gradient.

In one embodiment, the nucleic acids used in the method of the invention are fragmented in order to obtain a desired average fragment size. The skilled person will appreciate that the required length of nucleic acid fragment will depend on the sequencing technology that is used. For example, the Ion Torrent and MisSeq platforms utilise fragments from around 100 bp - 200 bp in length, whereas the Pacific Biosciences NGS platform can utilise nucleic acid fragments up to 20 kb in length. The nucleic acid may be fragmented by physical shearing, sonication, restriction digestion, or other suitable technique known in the art. The fragmenting of the nucleic acid can be performed so as to generate nucleic acid fragments having a desired average length. For example, the length, or the average length, of the nucleic acid fragments may be at least about 100 bp, at least about 200 bp, at least about 300 bp, at least about 400 bp, at least about 500 bp, at least about 600 bp, at least about 700 bp, at least about 800 bp, at least about 900 bp, at least about 1 kb, at least about 2 kb, at least about 3 kb, at least about 4 kb, at least about 5 kb, at least about 6 kb, at least about 7 kb, at least about 8 kb, at least about 9 kb, at least about 10 kb, at least about 11 kb, at least about 12 kb, at least about 15 kb, or at least about 20 kb.

In one embodiment, the nucleic acid sample is treated in order to generate single-stranded nucleic acid, or to generate nucleic acid comprising a single-stranded region, prior to contacting the sample with oligonucleotide probes. The nucleic acid can be made single-stranded using techniques known in the art, for example, including known hybridization techniques and commercial available kits such as the ReadyAmp™ Genomic DNA Purification System (Promega). Alternatively, single-stranded regions may be introduced into a nucleic acid using a suitable nickase in conjunction with an exonuclease.

### Oligonucleotide probes

The term "probe" or "oligonucleotide probe" according to the present invention refers to an oligonucleotide which is designed to specifically hybridize to a nucleic acid of interest. Preferably, the probes are suitable for use in preparing nucleic acid for high-throughput (next-gen) sequencing using a target capture technique. Thus, in one embodiment, the probe may be suitable for target capture and be around 60 to 120 nucleotides in length. Alternatively, the probe may be about 10 to 25 nucleotides. In certain embodiments, the length of the probe is 10, 11, 12, 13, 14, 15, 16, 17, 18, 19, 20, 21, 22, 23, 24 or 25 nucleotides. The oligonucleotide probes as used in the present invention may be ribonucleotides, deoxyribonucleotides and modified nucleotides such as inosine or nucleotides containing modified groups which do not essentially alter their hybridization characteristics.

Alternatively, the oligonucleotide probes may comprise nucleotide analogs. The term "nucleotide analogs" refers to synthetic analogs having modified nucleotide base portions, modified pentose portions, and/or modified phosphate portions, and, in the case of polynucleotides, modified internucleotide linkages, as generally described elsewhere (e.g., Scheit, Nucleotide Analogs, John Wiley, New York, (1980); Agarwal, Protocols for Polynucleotides and Analogs, Humana Press, (1994)). Generally, modified phosphate portions comprise analogs of phosphate wherein the phosphorous atom is in the +5 oxidation state and one or more of the oxygen atoms is replaced with a non-oxygen moiety, e.g., sulfur. Exemplary phosphate analogs include but are not limited to phosphorothioate, phosphorodithioate, phosphoroselenoate, phosphorodiselenoate, phosphoroanilothioate, phosphoranilidate, phosphoramidate, boronophosphates, including associated counterions, e.g., H⁺, NH₄⁺, Na⁺, if such counterions are present. Exemplary modified nucleotide base portions include but are not limited to 5-methylcytosine (5mC); C-5-propynyl analogs, including but not limited to, C-5 propynyl-C and C-5 propynyl-U; 2,6-diaminopurine, also known as 2-amino adenine or 2-amino-dA); hypoxanthine, pseudouridine, 2-thiopyrimidine, isocytosine (isoC), 5-methyl isoC, and isoguanine (isoG; see, e.g., U.S. Pat. No. 5,432,272). Exemplary modified pentose portions include but are not limited to, locked nucleic acid (LNA) analogs including without limitation Bz-A-LNA, 5-Me-Bz-C-LNA, dmf-G-LNA, and T-LNA, and 2'- or 3'-modifications where the 2'- or 3'-position is hydrogen, hydroxy, alkoxy (e.g., methoxy, ethoxy, allyloxy, isopropoxy, butoxy, isobutoxy and phenoxy), azido, amino, alkylamino, fluoro, chloro, or bromo. Modified internucleotide linkages include phosphate analogs, analogs having achiral and uncharged intersubunit linkages, and uncharged morpholino-based polymers having achiral intersubunit linkages (see, e.g., U.S. Pat. No. 5,034,506). Some internucleotide linkage analogs include morpholidate, acetal, and polyamide-linked heterocycles. In one class of nucleotide analogs, known as peptide nucleic acids, including pseudocomplementary peptide nucleic acids ("PNA"), a conventional sugar and internucleotide linkage has been replaced with a 2-aminoethylglycine amide backbone polymer.

### Hybridization

As used herein, the term "hybridization" refers to the process in which an oligonucleotide probe binds non-covalently with a target nucleic acid to form a stable double-stranded polynucleotide. Hybridization conditions will typically include salt concentrations of less than about 1 M, more usually less than about 500 mM and may be less than about 200 mM. A hybridization buffer includes a buffered salt solution such as 5% SSPE, or other such buffers known in the art. Hybridization temperatures can be as low as 5° C, but are typically greater than 22° C, and more typically greater than about 30° C, and typically in excess of 37° C. Hybridizations are usually performed under stringent conditions, i.e., conditions under which a probe will hybridize to its target sequence to which it is complementary, but will not hybridize to the other, non-complementary sequences.

As used herein the term "complementary" and grammatical equivalents refer to the nucleotide base-pairing interaction of one nucleic acid with another nucleic acid, including modified nucleic acids and nucleic acid analogues, that results in the formation of a duplex, triplex, or other higher-ordered structure. The primary interaction is typically nucleotide base specific, e.g., A:T, A:U, and G:C, by Watson-Crick and Hoogsteen-type hydrogen bonding. Conditions under which oligonucleotide probes anneal to complementary or substantially complementary regions of target nucleic acids well known in the art

As understood in the art., stringent hybridization conditions are sequence-dependent and are different in different circumstances. For example, longer fragments may require higher hybridization temperatures for specific hybridization than short fragments. As other factors may affect the stringency of hybridization, including base composition and length of the complementary strands, presence of organic solvents, and the extent of base mismatching, the combination of parameters is more important than the absolute measure of any one parameter alone. Generally stringent conditions are selected to be about 5° C lower than the Tₘ for the specific sequence at a defined ionic strength and pH. Example stringent conditions include a salt concentration of at least 0.01 M to no more than 1 M sodium ion concentration (or other salt) at a pH of about 7.0 to about 8.3 and a temperature of at least 25° C. For example, conditions of 5 × SSPE (750 mM NaCl, 50 mM sodium phosphate, 5 mM EDTA at pH 7.4) and a temperature of 30° C are suitable for allele-specific probe hybridizations.

### Capture tags and binding agents

In one embodiment, the oligonucleotide probes used in the method of the present invention comprise a capture tag to facilitate enrichment of a nucleic acid of interest bound to an oligonucleotide probe from other nucleic acid sequences in a sample. In order to enrich for the nucleic acid of interest from other nucleic acid sequences, the capture tag binds to a suitable binding agent. As would be understood in the art, the phrase "enriching for a nucleic acid" refers to increasing the amount of a target nucleic acid sequence in a sample relative to nucleic acid that is not bound to an oligonucleotide probe. Thereby, the ratio of target sequence relative to the corresponding non-target nucleic acid in a sample is increased.

In one embodiment, the capture tag is a "hybridization tag". As used herein, the term "hybridization tag" and grammatical equivalents can refer to a nucleic acid comprising a sequence complementary to at least a portion of a another nucleic acid sequence that acts as the binding agent (i.e., a "binding tag"). The degree of complementarity between a hybridization tag and a corresponding binding sequence can vary with the application. In some embodiments, the hybridization tag can be complementary or substantially complementary to a binding tag or portions thereof. For example, a hybridization tag can comprise a sequence having a complementarity to a corresponding binding tag of at least about 50%, at least about 60%, at least about 70%, at least about 80%, at least about 90%, and at least about 99%. In some embodiments, a hybridization tag can comprise a sequence having 100 % complementarity to a corresponding binding tag.

In some embodiments, a capture probe can include a plurality of hybridization tags for which the corresponding binding tags are located in the same nucleic acid, or different nucleic acids. In certain embodiments, hybridization tags comprising RNA may be advantageous to efficiently remove excess levels of such tags.

In certain embodiments, a hybridization tag can comprise at least about 5 nucleotides, at least about 10 nucleotides, at least about 15 nucleotides, at least about 20 nucleotides, at least about 25 nucleotides, at least about 30 nucleotides, at least about 35 nucleotides, at least about 40 nucleotides, at least about 45 nucleotides, at least about 50 nucleotides, at least about 55 nucleotides, at least about 60 nucleotides, at least about 65 nucleotides, at least about 70 nucleotides, at least about 75 nucleotides, at least about 80 nucleotides, at least about 85 nucleotides, at least about 90 nucleotides, at least about 95 nucleotides, and at least about 100 nucleotides,

In another embodiment, the capture tag may comprise an "affinity tag". As used herein, the term "affinity tag" and grammatical equivalents can refer to a component of a multi-component complex, wherein the components of the multi-component complex specifically interact with or bind to each other. For example an affinity tag can include biotin that can bind streptavidin. Other examples of multiple-component affinity tag complexes include, ligands and their receptors, for example, avidin-biotin, streptavidin-biotm, and derivatives of biotin, streptavidin, or avidin, including, but not limited to, 2-iminobiotin, desthiobiotin, NeutrAvidin (Molecular Probes, Eugene, Oreg.), CaptAvidin (Molecular Probes), and the like; binding proteins/peptides, including maltose-maltose binding protein (MBP), calcium-calcium binding protein/peptide (CBP); antigen-antibody, including epitope tags, including e-MYC, HA, and FLAG Tag™, and their corresponding anti-epitope antibodies; haptens, for example, dinitrophenyl and digoxigenin, and their corresponding antibodies; aptamers and their corresponding targets; fluorophores and anti-fluorophore antibodies; and the like. In one embodiment, affinity tags are used for the bulk separation of target nucleic acids comprising hybridization tags.

Thus, the binding agent used in the method of the invention is capable of binding an affinity tag as described herein to facilitate separation of a nucleic acid of interest from other nucleic acid sequences in a sample. For example, in one embodiment, the affinity tag comprises biotin and the binding agent comprises streptavidin. The binding agent is typically on a substrate. Examples of substrates include beads, microspheres, planar surfaces, columns, wells and the like. The terms "microsphere" or "bead" or "particle" or grammatical equivalents are understood in the art and refer to a small discrete particle. The composition of the substrate will vary on the application. Suitable compositions include those used in peptide, nucleic acid and organic moiety synthesis, including, but not limited to, plastics, ceramics, glass, polystyrene, methylstyrene, acrylic polymers, paramagnetic materials, thoria sol, carbon graphite, titanium dioxide, latex or cross-linked dextrans such as Sepbarose, cellulose, nylon, cross-linked micelles and Teflon may all be used "Microsphere Detection Guide" from Bangs Laboratories, Fishers Ind. is a helpful guide. The beads need not be spherical; irregular particles may be used. In some embodiments, a substrate can comprise a metallic composition, e.g., ferrous, and may also comprise magnetic properties. An example embodiment utilizing magnetic beads includes capture probes comprising streptavidin-coated magnetic beads. In addition, the beads may be porous, thus increasing the surface area of the bead available for association with capture probes. The bead sizes range from nanometers, i.e. 100 nm, to millimeters, i.e. 1 mm, with beads from about 0.2 µm to about 200 µm, or from about 0.5 to about 5 µm, although in some embodiments smaller beads may be used.

### High-throughput sequencing

The methods of the invention are particularly suited to preparing nucleic acid for sequencing using a high-throughput sequencing technology such as Next-generation sequencing technology. Next-generation sequencing (NGS) technologies include instruments that are capable of sequencing more than 10¹⁴ kilobase-pairs (kbp) of DNA per instrument run. Sequencing typically produces a large number of independent reads, each representing anywhere between 10 to 1000 bases of the nucleic acid. Nucleic acids are generally sequenced redundantly for confidence, with replicates per unit area being referred to as the coverage (i.e., "10 × coverage" or "100 × coverage"). Next generation sequencing methods are known in the art, and are described, for example, in Metzker (2010).

Thus, the terms "Next-generation sequencing" or "NGS" or "NG sequencing" as used herein refer to any sequencing method that determines the nucleotide sequence of either individual nucleic acid molecules (e.g., in single molecule sequencing) or clonally expanded proxies for individual nucleic acid molecules in a high through-put fashion (e.g., greater than 10³, 10⁴, 10⁵ or more molecules are sequenced simultaneously).

Platforms for next-generation sequencing include, but are not limited to, Roche/454's Genome Sequencer (GS) FLX System, Illumina/Solexa's Genome Analyzer (GA), Life/APG's Support Oligonucleotide Ligation Detection (SOLiD) system, Polonator's G.007 system, Helicos BioSciences' HeliScope Gene Sequencing system, and Pacific Biosciences' PacBio RS system.

### Highly Polymorphic Genes and HLA Typing

As used herein, the term "highly polymorphic gene" includes reference to genes that have greater levels of polymorphism in the coding region of the gene compared to the non-coding regions. For example, a highly polymorphic gene may have a greater number of polymorphisms per kb of coding sequence when compared to the number of polymorphisms per kb of non-coding sequence of the gene. Well known examples of highly polymorphic genes are the human leukocyte antigen (HLA) genes.

The coding regions of HLA molecules are highly polymorphic as it is thought they are under positive select pressure to evolve in response to pathogenic threat. The non-coding regions of HLA are not under such selective pressure and do not share the same degree of polymorphism. While the non-coding regions of HLA class I are polymorphic, the polymorphisms are not randomly distributed across these regions and closely related, by coding sequence similarity, have identical non coding sequences.

Analysis of sequence alignments from non-coding regions of class I HLA reveal a limited set of unique sequences. The present inventors have determined that it is possible to design probes to each of the set of unique sequences to capture DNA fragments that can then be used in Next Gen sequencing assays.

Therefore, instead of an approach that utilizes overlapping probes from an exome (i.e. probes designed in exon regions) the present inventors describe the use of probes designed explicitly to the non-coding regions of HLA. Figure 1 shows a sequence alignment of HLA-A intron 1 demonstrating that different HLA alleles have identical and predictable HLA sequence. HLA allele nomenclature utilizes up to four fields separated by a colon (eg A^{∗}01:01:01:01). The first field represents, in general a amino acid sequence differences that can be detected by antibodies. The second field represents nucleotide differences that result in amino acid changes but may not be detectable with the use of antibodies. The third field represent synonymous changes and the forth field represents changes in non-coding regions. The use of letter code suffixes, such as the N in A^{∗}01:01:01:02N, indicates that the allele is an expression variant. For example N = Null or not expressed. As can be seen all A^{∗}01 alleles have identical sequence, except for 2 (A^{∗}01:02 and A^{∗}01:20) that share a nucleotide with A^{∗}30 alleles, and A^{∗}03 and A^{∗}11 alleles are also identical to the majority of A^{∗}01 alleles. Given that a probe will equally capture DNA that have a single base pair mismatch, a single probe should capture all A^{∗}01, A03, A^{∗}11 and A^{∗}30 alleles.

In one embodiment, the methods of the present invention comprise identifying alleles in one or more HLA-A, HLA-B, HLA-C, HLA-DPA1, HLA-DPB1, HLA-DQA1, HLA-DQB1, HLA-DRA, and/or HLA-DRB1 genes.

In some embodiments, the methods of the invention are useful in determining suitable donors for transplant recipients, and/or for determining the likelihood of development of graft versus host disease (GVHD) or acute GVHD (aGVHD) in a patient by matching gene alleles, for example HLA gene alleles, in both the transplant donor and recipient.

In addition, determination of a likelihood of developing aGVHD in a transplant candidate may influence the determination as to whether transplantation is indeed the most suitable form of treatment for their condition. In some instances, there may be alternate forms of therapy available which would provide a better prognosis to the patient. Also, prediction of a likelihood of aGVHD would be indicative of the necessity for treatment regimes, or possibly more aggressive treatment regimes than would otherwise be recommended. Such aggressive therapies often have undesireable side effects and so preferably are not used unless prognosis indicates a need. For example, treatment of patients with neutralizing anti-TNF-alpha monoclonal antibodies can result in amelioration of aGVHD, but can increase risk of infections. Various aggressive anti-inflammatory therapies are also available.

As used herein, the terms "transplant" or "transplanting" refer to the grafting or introduction of tissue or cells obtained from one individual (the donor) into or onto the body of another individual (the recipient). The cells or tissue that are removed from the donor and transplanted into the recipient are referred to as a "graft". Examples of tissues commonly transplanted are bone marrow, hematopoetic stem cells, organs such as liver, heart, skin, bladder, lung, kidney, cornea, pancreas, pancreatic islets, brain tissue, bone, and intestine. The person skilled in the art would understand that the term "haplotype" refers to a combination of alleles that are located closely, or at adjacent loci, on a chromosome and that are inherited together, or a set of single nucleotide polymorphisms on a single chromosome of a chromosome pair that are statistically associated.

### Kits

Also described herein is a kit for identifying HLA gene alleles according to the method of the invention. The kit contains oligonucleotide probes that are able to hybridize to HLA gene target sequences in a non-coding region of the gene. The oligonucleotide probes may comprise a capture tag such as, for example, biotin or streptavidin. The kits may further comprise a binding agent capable of binding the capture tag. The binding agent may be coated on or attached to a suitable substrate such as, for example, a microsphere or bead. The substrate may be magnetic to facilitate enrichment of a target nucleic acid of interest.

### EXAMPLES

### Example 1. DNA Library Preparation

Illumina libraries were prepared from 200ng genomic DNA using the Illumina TruSeq DNA Nano protocol, selecting for target inserts of 550bp in size.

### Example. 2. HLA Capture Using Intron-Specific Probes

As proof of concept, a custom oligonucleotide probe pool (SEQ ID NOs:1 to 8) targeted to intron sequences was diluted to a concentration of 1.5pmol/µl. For a first hybridization procedure, Human Cot DNA, amplified library (prepared as above), universal blocking oligo TS-p7(6nt), and universal blocking oligo TS-p7(8nt) were added to a tube and dried down in a vacuum concentrator set at 60° C for approximately 1 hour. Dried down sample was then re-suspended in Nimblegen 2X Hybridisation buffer, Nimblegen Hybridisation Component A and nuclease free water. Resuspended sample was then vortexed and denatured at 95°C for 10 minutes. Following denaturation, 2µl of probe (3pmol) was added to the sample and incubated at 72°C for 4 hours.

To wash and recover probe-hybridized DNA, wash solutions from the Nimblegen wash kits were diluted according to the manufacturers protocol. The hybridized sample was combined with streptavidin beads, vortexed and incubated at 72°C for 45 minutes. After incubation, bound DNA was washed with wash I and stringent wash at 72°C, followed by wash II and III at room temperature. The hybridised sample bound to the streptavidin beads was then placed on a magnet and the bound DNA was eluted off using 0.125N NaOH. The hybridised sample was then purified using Ampure XP beads and then amplified for 12 cycles with KAPA HiFi HotStart ReadyMix. The primers used in the PCR were complementary to the Illumina adaptor and barcode sequences in the ligated DNA (Illumina P5 and P7). The sample was purified with Ampure XP beads.

### Example 3. Sequencing of Hybridized DNA

The amplified hybridized sample was sequenced on a MiSeq or HiSeq using Illumina 2x300bp sequencing protocol.

### Example 4. Results

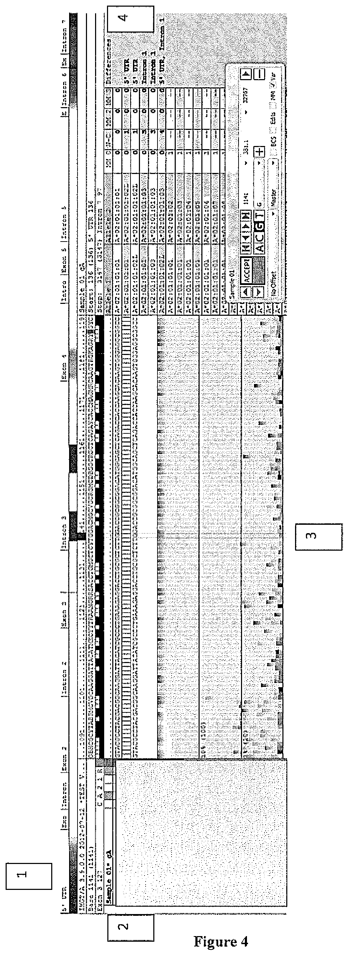

The sequence data generated was analysed with Assign MPS v 1.0 sequence analysis software (Conexio Genomics, Fremantle, Australia). Analysis of sequence data generated using a targeted capture sequencing protocol is shown in Figure 4. Using the Assign MPS v 1.0 software, the best matched genotype for the sample was determined to be HLA-A *02:01:01:01+A*02:01:01:01.

### Example 5. Next Generation Sequencing Following Capture Probe Enrichment of 3 Samples also Sequenced by Sanger

The Sanger assay amplifies HLA-A, -B and -C sequence in amplicons that span the 5' untranslated region to the end of intron 4 for HLA-A and -B and the 5' untranslated region to the 3' untranslated of HLA-C. All heterozygous ambiguities had been resolved using allele specific sequencing primers.

The Capture Probe assay was performed using the probes described in Figure 3 and provided as SEQ ID NOs:10-71, and with the protocol described in the text above. For the 3 samples there was complete concordance for the reported genotypes. The results of the assay are shown in Figure 5.

Sample 1 HLA-B contains an allele for which a reference sequence has not been reported. This allele has a single mismatch at base 444 of compared with the sequence of B^{∗}15:01:01:01, located in intron 1. By locus specific amplification using Sanger, this sample requires an additional experiment using B^{∗}08:01:01 and B^{∗}15:01:01 allele specific sequencing primers to determine of the new allele is an undescribed B^{∗}08-01:01 allele of B^{∗}15:01:01:01 allele. NGS enables phasing of polymorphisms throughout the gene and can readily identify that the new allele is a B^{∗}15:01:01 subtype. This sample also contains a novel C^{∗}04:01:01 allele. The polymorphism is located in intron 5. The use of allele specific *sequencing* primers are not commonly used in this region and characterisation by Sanger sequencing would require the alleles to be separated by PCR, followed by Sanger sequencing. However, NGS correctly identified the new allele to be a C^{∗}04:01:01 subtype.

This data demonstrates that the use of Capture Probes enable the identification of new alleles and also the ability of NGS to determine the phase relationship of sequence polymorphisms, thus enabling the precise characterisation of the new allele.

### REFERENCES

Metzker (2010) Nat Rev Genet, 11(1):31-46

## Claims

1. A method for identifying one or more HLA gene alleles in a subject, the method comprising:
a) contacting a nucleic acid sample from the subject with oligonucleotide probes wherein one or more of the oligonucleotide probes hybridize to HLA gene target sequences in the nucleic acid sample;
b) enriching for a nucleic acid that is hybridized to an oligonucleotide probe by separating it from nucleic acid not bound to an oligonucleotide probe; and
c) sequencing the enriched nucleic acid by high-throughput screening to identify one or more HLA gene alleles;
wherein the gene target sequences are in a non-coding region of one or more HLA genes, and
wherein the oligonucleotide probes are suitable for preparing the nucleic acid for high-throughput screening using target capture.

2. The method of claim 1, wherein the oligonucleotide probes comprise a capture tag.

3. The method of any one of the preceding claims, wherein the nucleic acid sample contacted with the oligonucleotide probes comprises single stranded nucleic acid.

4. The method of any one of claims 1 to 3, wherein the nucleic acid sample is fragmented before being contacted with the oligonucleotide probes.

5. The method of claim 1 further comprising fragmenting the nucleic acid sample to obtain nucleic acid fragments of at least about 2 kb in length prior to step (a).

6. The method according to any one of the preceding claims, wherein one or more of the oligonucleotide probes comprises a nucleic acid sequence at least 95% identical to any one of SEQ ID Nos:1-8 or 10-71.

7. A method of identifying a transplant donor for a recipient in need of a transplant, the method comprising:
a) performing the method of any one of the preceding claims to identify one or more HLA gene alleles in the recipient in need of a transplant; and
b) identifying a transplant donor based on the presence of one or more alleles in an HLA gene of both the transplant donor and the transplant recipient.

8. A method of reducing the likelihood of a transplant recipient developing graft versus host disease, the method comprising:
a) performing the method of any one of the proceeding claims to identify one or more HLA gene alleles in the recipient in need of a transplant; and
b) identifying a transplant donor based on the presence of one or more HLA gene alleles in both the transplant donor and the transplant recipient;
wherein the presence of the one or more HLA gene alleles in both the transplant recipient and the transplant donor is indicative of a reduced likelihood of the transplant recipient developing graft versus host disease following transplantation of a graft from the transplant donor.

## Patentansprüche

1. Verfahren zur Identifizierung eines oder mehrerer HLA-Genallele in einem Subjekt, wobei das Verfahren umfasst:
a) Inkontaktbringen einer Nukleinsäureprobe aus dem Subjekt mit Oligonukleotidsonden, wobei eine oder mehrere der Oligonukleotidsonden mit HLA-Gen-Zielsequenzen in der Nukleinsäureprobe hybridisieren;
b) Anreichern auf eine Nukleinsäure, die an eine Oligonukleotidsonde hybridisiert ist, durch Abtrennen dieser von nicht an eine Oligonukleotidsonde gebundener Nukleinsäure; und
c) Sequenzieren der angereicherten Nukleinsäure durch Hochdurchsatz-Screening, um ein oder mehrere HLA-Genallele zu identifizieren;
wobei die Gen-Zielsequenzen in einer nicht-kodierenden Region eines oder mehrerer HLA-Gene liegen, und
wobei die Oligonukleotidsonden geeignet sind, die Nukleinsäure für Hochdurchsatz-Screening unter Verwendung von Zieleinfang (target capture) vorzubereiten.

2. Verfahren nach Anspruch 1, bei dem die Oligonukleotidsonden eine Einfangmarkierung aufweisen.

3. Verfahren nach einem der vorhergehenden Ansprüche, bei dem die mit den Oligonukleotidsonden in Kontakt gebrachte Nukleinsäureprobe einzelsträngige Nukleinsäure umfasst.

4. Verfahren nach einem der Ansprüche 1 bis 3, bei dem die Nukleinsäureprobe fragmentiert wird, bevor sie mit den Oligonukleotidsonden in Kontakt gebracht wird.

5. Verfahren nach Anspruch 1, umfassend ferner das Fragmentieren der Nukleinsäureprobe, um vor Schritt (a) Nukleinsäurefragmente von mindestens etwa 2 kb Länge zu erhalten.

6. Verfahren nach einem der vorhergehenden Ansprüche, bei dem eine oder mehrere der Oligonukleotidsonden eine Nukleinsäuresequenz umfassen, die zu mindestens 95% identisch ist mit einer der Sequenzen SEQ ID Nr. 1-8 oder 10-71.

7. Verfahren zum Identifizieren eines Transplantatspenders für einen Empfänger, der ein Transplantat benötigt, wobei das Verfahren umfasst:
a) Durchführen des Verfahrens nach einem der vorhergehenden Ansprüche, im ein oder mehrere HLA-Gen-Allelele bei dem ein Transplantat benötigenden Empfänger zu identifizieren; und
b) Identifizieren eines Transplantatspenders aufgrund des Vorhandenseins eines oder mehrerer Allele in einem HLA-Gen sowohl des Transplantatspenders als auch des Transplantatempfängers.

8. Verfahren zur Verringerung der Wahrscheinlichkeit, dass ein Transplantatempfänger eine Graft-versus-Host-Krankheit entwickelt, wobei das Verfahren umfasst:
a) Durchführen des Verfahrens nach einem der vorhergehenden Ansprüche, um ein oder mehrere HLA-GenAllele in dem Empfänger zu identifizieren, der ein Transplantat benötigt; und
b) Identifizieren eines Transplantatspenders aufgrund des Vorhandenseins eines oder mehrerer HLA-Genallele sowohl beim Transplantatspender als auch beim Transplantatempfänger;
wobei das Vorhandensein eines oder mehrerer HLA-GenAllele sowohl beim Transplantatempfänger als auch beim Transplantatspender auf eine verringerte Wahrscheinlichkeit hindeutet, dass der Transplantatempfänger nach der Transplantation eines Transplantats vom Transplantatspender eine Graft-versus-Host-Krankheit entwickelt.

## Revendications

1. Procédé pour identifier un ou plusieurs allèles de gène HLA chez un sujet, le procédé comprenant les étapes consistant à :
a) mettre en contact un échantillon d'acide nucléique provenant du sujet avec des sondes oligonucléotidiques, une ou plusieurs des sondes oligonucléotidiques s'hybridant à des séquences cibles de gène HLA dans l'échantillon d'acide nucléique ;
b) enrichir un acide nucléique qui est hybridé à une sonde oligonucléotidique en le séparant d'un acide nucléique non lié à une sonde oligonucléotidique ; et
c) séquencer l'acide nucléique enrichi par criblage à haut débit pour identifier un ou plusieurs allèles de gène HLA ;
dans lequel les séquences cibles de gène sont dans une région non codante d'un ou plusieurs gènes HLA, et
dans lequel les sondes oligonucléotidiques sont appropriées pour préparer l'acide nucléique pour un criblage à haut débit en utilisant une capture de cible.

2. Procédé selon la revendication 1, dans lequel les sondes oligonucléotidiques comprennent une étiquette de capture.

3. Procédé selon l'une quelconque des revendications précédentes, dans lequel l'échantillon d'acide nucléique mis en contact avec les sondes oligonucléotidiques comprend un acide nucléique simple brin.

4. Procédé selon l'une quelconque des revendications 1 à 3, dans lequel l'échantillon d'acide nucléique est fragmenté avant d'être mis en contact avec les sondes oligonucléotidiques.

5. Procédé selon la revendication 1, comprenant en outre la fragmentation de l'échantillon d'acide nucléique pour obtenir des fragments d'acide nucléique d'au moins environ 2 kb de longueur avant l'étape (a).

6. Procédé selon l'un quelconque des revendications précédentes, dans lequel une ou plusieurs des sondes oligonucléotidiques comprend une séquence d'acide nucléique identique à au moins 95 % avec l'une quelconque des SEQ ID No : 1 à 8 ou 10 à 71.

7. Procédé d'identification d'un donneur de transplantation pour un receveur nécessitant une transplantation, le procédé comprenant :
a) la mise en œuvre du procédé selon l'une quelconque des revendications précédentes pour identifier un ou plusieurs allèles de gène HLA chez le receveur nécessitant une transplantation ; et
b) l'identification d'un donneur de transplantation sur la base de la présence d'un ou plusieurs allèles dans un gène HLA à la fois du donneur de transplantation et du receveur de transplantation.

8. Procédé pour réduire la probabilité qu'un receveur de transplantation développe une maladie du greffon contre l'hôte, le procédé comprenant :
a) la mise en œuvre du procédé selon l'une quelconque des revendications précédentes pour identifier un ou plusieurs allèles de gène HLA chez le receveur nécessitant une transplantation ; et
b) l'identification d'un donneur de transplantation sur la base de la présence d'un ou plusieurs allèles de gène HLA à la fois chez le donneur de transplantation et le receveur de transplantation ;
dans lequel la présence des un ou plusieurs allèles de gène HLA à la fois chez le receveur de transplantation et chez le donneur de transplantation est représentative d'une probabilité réduite que le receveur de transplantation développe une maladie du greffon contre l'hôte après transplantation d'un greffon depuis le donneur de transplantation.
